(19) Europäisches Patentamt
European Patent Office
Office européen des brevets

(11) **EP 3 501 577 A1**

(12) **EUROPÄISCHE PATENTANMELDUNG**

(43) Veröffentlichungstag:
**26.06.2019  Patentblatt 2019/26**

(51) Int Cl.:
**A61M 5/32** (2006.01)  **A61M 5/142** (2006.01)
**A61M 5/158** (2006.01)

(21) Anmeldenummer: **17209764.4**

(22) Anmeldetag: **21.12.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **TecPharma Licensing AG
3401 Burgdorf (CH)**

(72) Erfinder:
• **BURI, Thomas**
  **3400 Burgdorf (CH)**
• **HOFER, Christophe**
  **3400 Burgdorf (CH)**
• **HOSTETTLER, Patrick**
  **3415 Hasle (CH)**
• **STAUB, Seline**
  **8604 Volketswil (CH)**
• **STECK, Jürg**
  **3422 Kirchberg (CH)**
• **STREIT, Ursina**
  **3322 Schönbühl (CH)**

(54) **KANÜLENINSERTIONSMECHANISMUS**

(57) Die Erfindung betrifft einen Insertionsmechanismus für eine Kanüle (7, 8) mit: einem Kanülengehäuse (1), relativ zu welchem die Kanüle (7, 8) verschoben werden kann; mindestens einem Kanülenhalter (2.2, 3.2), an welchem die Kanüle (7, 8) befestigbar oder befestigt ist und welcher relativ zum Kanülengehäuse (1) bewegbar ist; einem Insertions-Energiespeicher (4) zum Erzeugen einer Insertionskraft auf den mindestens einen Kanülenhalter (2.2, 3.2) und zum Erzeugen einer Freigabekraft auf ein Halteelement (1.1); einem Halteelement (1.1), welcher den mindestens einen Kanülenhalter (2.2, 3.2) gegen die vom Insertions-Energiespeicher (4) erzeugte Kraft relativ zum Kanülengehäuse (1) halten kann; und einer Sicherung (9), welche das Halteelement (1.1) im Sicherungszustand gegen eine Kraft des Insertions-Energiespeichers (4) in Sperrstellung hält und im ausgelösten Zustand das Halteelement (1.1) freigibt, sodass das Halteelement (1.1) durch die Kraft des Insertions-Energiespeichers (4) in Freigabestellung bewegt werden kann und sich der mindestens eine Kanülenhalter (2.2, 3.2) angetrieben durch den Insertions-Energiespeicher (4) relativ zum Kanülengehäuse (1) bewegen kann, um eine Insertionsbewegung oder Insertion auszulösen oder durchzuführen.

Fig. 1A

Fig. 1B

**Beschreibung**

Die vorliegende Erfindung betrifft eine Vorrichtung zur Verabreichung einer Substanz, zum Beispiel einer flüssigen medizinischen Substanz, welche mittels einer Kanüle, die in einen Körper eingebracht wird, abgegeben wird. Insbesondere betrifft die Erfindung einen Mechanismus zur automatischen Insertion einer Kanüle, mit welchem eine Kanüle automatisch in einen Körper eingestochen werden kann, indem diese zum Beispiel aus einer Verabreichungsvorrichtung oder einem Kanülengehäuse ausgestoßen wird. Nach dem Eindringen der Kanüle in einen Körper kann eine medizinische Substanz, wie zum Beispiel Insulin, durch die Kanüle dosiert an den Körper abgegeben werden.

Zur Verabreichung einer medizinischen Substanz oder Flüssigkeit wird häufig eine an sich bekannte Spritze, ein Injektionspen oder eine Infusionspumpe verwendet. Die Infusionspumpe kann an ein Infusionsset oder einen Katheter angeschlossen werden, um die Flüssigkeit dosiert abzugeben. Eine Möglichkeit der kontrollierten Substanzverabreichung ist die Abgabe durch eine externe Pumpe an einen Patienten, welche zum Beispiel an dem Patienten getragen oder auf dessen Haut aufgeklebt wird, wobei die abzugebende Substanz aus der Pumpe durch einen in den Körper des Patienten eingebrachten Katheter abgegeben wird.

Das Einbringen eines Katheters oder Kanüle kann manuell, zum Beispiel mittels eines an sich bekannten Infusionssets, erfolgen. Alternativ zum manuellen Setzen einer Kanüle ist auch eine automatische Kanüleneinbringvorrichtung bekannt.

Die US 9,220,838 B2 offenbart eine Kanüleneinbringvorrichtung, bei welcher eine Kanüle in einem Gehäuse durch eine erste Feder vorgespannt ist, wobei die Feder durch ein Rückhalteteil an der Entspannung und dem Vorschub der Kanüle gehindert wird. Das Rückhalteteil ist ein drehbarer Hebel, der seinerseits von einer weiteren zweiten Feder in Freigaberichtung mit einer Kraft beaufschlagt wird. Das drehbare Rückhalteteil wird durch einen Aktuator gegen die Kraft der weiteren Feder in Blockierstellung gehalten, wobei nach Betätigung des Aktuators und Freigabe des Rückhalteteils das Rückhalteteil durch die weitere zweite Feder aus der Blockierposition in eine Freigabeposition gezogen wird, sodass die erste Feder sich entspannen kann und die Kanüleninsertion bewirken kann. In einer Ausführungsform kann der Aktuator zum Rückhalten des Sperrelements gegen die Kraft der zweiten Feder ein Hakenelement sein, welches mit einem Schmelzteil verbunden ist, das von einem Draht, z.B. in Form einer Heizwendel, umwickelt ist. Fließt ein elektrischer Strom durch den Draht, schmilzt der Schmelzteil, sodass das Sperrelement bedingt durch die Kraft der zweiten Feder um eine Drehachse gedreht wird und die erste Feder funktional freigibt.

Ausführungsformen der erfindungsgemäßen Vorrichtung können einen oder mehrere der folgenden Vorteile bieten: Eine sichere Lagerung, insbesondere eine sichere Langzeitlagerung der Vorrichtung. Eine sichere Auslösung der Insertion, insbesondere bei durch langzeitigen Druck oder Zug beaufschlagten Komponenten. Immunität bezüglich Beschleunigungen, wie sie z.B. bei einem Fall oder Stoß auftreten können. Eine einfache automatische z.B. durch ein elektrisches Signal ausgelöste Ansteuerung, wie z.B. eine Auslösung einer Insertion. Erhöhung der Montageprozesssicherheit. Verringerung des für den Halte- oder Auslösemechanismus benötigten Platzbedarfs.

Sofern nachfolgend auf eine Kanüle Bezug genommen wird, ist hierunter sowohl eine einzelne Kanüle, also beispielsweise eine Softkanüle, oder alternativ auch ein Mehr-Elemente-System zu verstehen, wie beispielsweise eine Hartkanüle, Stahlkanüle oder Nadel, welche von zum Beispiel einer Softkanüle (z.B. aus elastischem Kunststoff) umgeben wird. In letzterem Fall kann bei der Insertion beispielsweise die die Hartkanüle umgebende Softkanüle zusammen mit dieser in ein Gewebe eingestochen werden und nach der Insertion kann die relativ zur Softkanüle verschiebbare Hartkanüle innerhalb der Softkanüle zurück und beispielsweise aus dem Gewebe herausgezogen werden, wobei die Softkanüle in dem Gewebe bleibt. Ein Fluid, welches beispielsweise an dem Einstichort (= distales Ende) abgewandten Ende der Hartkanüle (proximal) in diese eintritt, kann durch die Hartkanüle hindurchtreten und tritt an der distalen Austrittsöffnung der Hartkanüle in den Innenraum der die Hartkanüle umgebenden Softkanüle ein und wird von der Softkanüle weitergeleitet, bis es am distalen Ende der Softkanüle abgegeben wird. Die Softkanüle liegt mit ihrem Innenumfang dichtend am Außenumfang der Hartkanüle an, sodass keine Flüssigkeit durch den Bereich hindurchtreten kann, an welchem sich Hartkanüle und Softkanüle berühren. Dabei ist die Hartkanüle vorzugsweise innerhalb der Softkanüle, welche beispielsweise aus einem Kunststoff oder verformbaren Material geformt ist, verschiebbar. Beide, Soft- und Hartkanüle, sind bevorzugt biegbar, also können z.B. in ihrer Längsrichtung gebogen oder gekrümmt werden. Alternativ kann, bei ansonsten gleicher und dichtender Ausgestaltung, die Hartkanüle außerhalb der Softkanüle angeordnet sein.

Gemäß einem ersten Aspekt betrifft die Erfindung einen Insertionsmechanismus für eine Kanüle, die beispielsweise als Softkanüle oder wie oben erwähnt als Hartkanüle mit umgebender Softkanüle oder nur als Hartkanüle ausgebildet sein kann. Die Kanüle ist verschiebbar relativ zu einem Kanülengehäuse gelagert, sodass die Kanüle beispielsweise relativ zu dem Kanülengehäuse beschleunigt oder z.B. aus dem Kanülengehäuse automatisch ausgefahren oder ausgestoßen werden kann. Das Kanülengehäuse kann eine die Kanüle teilweise oder vollständig umgebende Struktur sein, welche eine oder eine Mehrzahl von Öffnungen aufweisen kann, wobei die Kanüle beispielsweise auch vollständig oder zum Teil außerhalb des Kanülengehäuses geführt werden kann. Die Kanüle ist an mindestens einem Kanülenhalter befestigt, welcher relativ zum Kanülengehäuse verschoben werden kann, um so einen Ausschub oder Einstich der Kanüle zu bewirken. Der oder jeder Kanülenhalter ist relativ zum Kanülengehäuse und beispielsweise im Kanülengehäuse beweglich und kann beispielsweise in eine Kanülenvorschubrichtung und eine Kanülenrückzugsrichtung verschoben werden, wobei die Einstichrichtung der Kanüle auf Grund einer Kanülenkrümmung auch von der Kanülenvorschubrichtung oder Kanülenrückzugsrichtung abweichen oder davon verschieden sein kann. Die mit dem mindestens einen Kanülenhalter verbundene mindestens eine Kanüle kann in distaler axialer Richtung relativ zum Haltepunkt an dem Kanülenhalter geradlinig weiterverlaufen oder geführt sein und kann auch eine oder mehrere Krümmungen oder Biegungen aufweisen, sodass die Kanüle beispielsweise entlang oder in Fortsetzung eines Verschiebeweges des Kanülenhalters z.B. entlang des Kanülengehäuses geradlinig geführt und im weiteren Verlauf gekrümmt und anschließend wieder geradlinig geführt ist. Mindestens ein Energiespeicherelement zum Erzeugen einer Insertionsbewegung des Kanülenhalters und damit zum Erzeugen einer Insertionsbewegung der Kanüle kann den Kanülenhalter mit einer Kraft in Richtung Vorschub- oder der Insertionsbewegung beaufschlagen, um nach Auslösung des Energiespeicherelements eine automatische Insertion durchführen zu können. Das Energiespeicherelement kann beispielsweise mindestens eine vorgespannte Druckfeder, z.B. auch mehrere parallel oder seriell angeordnete Federn, oder alternativ auch eine oder mehrere Zugfedern sein, welche beispielsweise funktional zwischen dem Kanülengehäuse und dem Kanülenhalter vorgesehen ist und optional mit einem oder beiden dieser Elemente auch verbunden sein kann. Das Energiespeicherelement kann auch ein anderes mechanisches oder hydraulisches oder pneumatisches Element oder ein magnetischer, elektromagnetischer oder elektromechanischer Antrieb sein, welcher den Kanülenhalter relativ zum Kanülengehäuse bewegen kann.

Der Energiespeicher wird von einem Sperr- oder Rückhalteelement an seiner Entladung oder Entspannung oder an der Freisetzung der permanent wirkenden Kraft gehindert, wobei das Sperr- oder Rückhalteelement unmittelbar auf den Energiespeicher oder mittelbar auf den Energiespeicher, zum Beispiel auf den Kanülenhalter, wirken kann, um den Energiespeicher oder Kanülenhalter im gesperrten Zustand an einer Bewegung in Insertionsrichtung zu hindern. Das Sperr- oder Halteelement kann als einzelnes Element, z.B. als Federarm, oder zusammengesetzt aus zwei oder mehr Teilelementen, z.B. zwei oder mehr Federarmen, ausgebildet sein, welche gemeinsam und/oder jeweils einzeln den Insertions-Energiespeicher sichern oder sperren, also an einer Entladung und Energieübertragung hindern, können. Das Sperr- oder Halteelement kann auch eine mechanische Übersetzung oder Untersetzung aufweisen und z.B. als Reduktionsgetriebe zur Reduktion oder Verringerung der zu haltenden Kraft ausgebildet sein. Dabei kann das Reduktionsgetriebe einstufig, zweistufig oder mehrstufig ausgebildet sein und z.B. Zahnräder und/oder Gewindeelemente zur Realisierung der Untersetzung aufweisen. Ein z.B. schmelzbares, erweichbares oder schwächbares Sicherungselement zum Sichern des Sperr- oder Halteelements kann ebenfalls zwei oder mehrfach vorgesehen sein, um die entsprechende Anzahl an Sperrelementen zu sichern. Die Erweichung oder Schwächung kann z.B. durch Temperaturerhöhung herbeigeführt werden. Das Material muss nicht zwingend flüssig werden. Zum Beispiel geht beim Überschreiten der Glasübergangstemperatur Tg ein fester amorpher Stoff oder festes Glas oder Polymer in einen gummiartigen bis zähflüssigen Zustand über, wodurch die Sperr- oder Haltefunktion aufgehoben werden kann.

Dabei wird gemäß einer optionalen Ausführungsform beispielsweise das Sperr- oder Rückhalteelement von dem Insertions-Energiespeicher permanent mit einer Kraft beaufschlagt, die das Rückhalteelement in Freigabestellung drückt, sodass das Rückhalteelement beispielsweise von einer Sicherung in der Sperrstellung gehalten werden muss, um die Insertion nicht auszulösen. Beispielsweise kann das Rückhalteelement oder Halteelement ein Hebel oder allgemein eine Struktur sein, auf welche die Kraft des Insertions-Energiespeichers im gesicherten und nicht ausgelösten Zustand wirkt, wobei die Sicherung ohne einen weiteren Antrieb, also zum Beispiel ohne eine weitere Feder, allein durch die Kraft des Insertions-Energiespeichers, wie beispielsweise einer Insertionsfeder, aus der Sperrposition weggedrückt werden könnte, um so den Kanülen-Insertionsvorgang freizugeben oder auszulösen, wenn das Rückhalteelement oder Halteelement nicht durch die Sicherung gesichert wäre, welche das Halteelement in Sperrposition hält. Der Insertionsvorgang kann somit beispielsweise mit nur einem einzigen Energiespeicher, wie beispielsweise einer einzigen Insertionsfeder, durchgeführt werden, welche einerseits die Energie für die Insertion, also beispielsweise den Vorschub der mindestens einen Kanüle, zur Verfügung stellt und welche andererseits die Energie zur Verfügung stellt, um das Halteelement aus der Halteposition wegzudrücken, nachdem die Sicherung das Halteelement freigegeben hat, also beispielsweise eine Bewegung des Halteelementes aus der Sperrstellung in eine Freigabestellung durch den Druck der Insertionsfeder ermöglicht hat. Alternativ kann das Sperr- oder Halteelement auch nicht vom Insertions-Energiespeicher mit einer Kraft beaufschlagt werden. Optional kann hierzu z.B. ein weiterer Energiespeicher oder eine weitere Feder vorgesehen sein, wie z.B. in der US 9,220,838 B2 beschrieben.

Das Sicherungselement, welches das Halteelement oder Auslöseelement des Insertions-Energiespeichers in Sperrstellung hält und zum Beispiel dessen Auslenkung zur Freigabe oder Durchführung der Insertion verhindert, kann gemäß einer Ausführungsform ein Element aus einem schmelzbaren, erweichbaren oder schwächbaren Material, wie zum Beispiel ein Kunststoffelement oder ein festes Glas oder Polymer sein, welches zum Beispiel elektrisch nicht leitfähig oder alternativ auch elektrisch leitfähig sein kann. Das feste Glas, der feste amorphe Stoff oder das Polymer kann in einen gummiartigen bis zähflüssigen Zustand übergehen, wenn die Glasübergangstemperatur Tg überschritten wird. Eine Schmelztemperatur muss nicht zwingend erreicht werden. Das schmelzbare, erweichbare oder schwächbare Element kann gemäß einer Ausführungsform auf Zug belastet sein, wobei die Zugkraft beispielsweise durch den Insertions-Energiespeicher verursacht wird, welcher direkt oder mittelbar auf das Halteelement drückt, welches mit der Sicherung oder dem schmelzbaren, erweichbaren oder schwächbaren Element gekoppelt oder verbunden ist.

Das mindestens eine Sicherungselement kann stoffschlüssig und/oder formschlüssig mit dem oder den Halteelementen oder Auslöseelementen verbunden sein. Eine stoffschlüssige Verbindung ermöglicht einen einfachen und sicheren Aufbau sowie ein sicheres langfristiges Halten des Insertions-Energiespeichers.

Am aufweichbaren oder schmelzbaren Element oder in dessen Nähe ist mindestens ein Erwärmungs- oder Heizelement vorgesehen, wie zum Beispiel ein Heizdraht, Heizwendel oder eine Heizfläche, welches z.B. bei Stromdurchfluss Wärme erzeugt, um das schmelzbare Element durch Erwärmung zumindest teilweise oder vollständig aufweichen oder schmelzen zu können. Wenn das schmelzbare oder aufweichbare Element bedingt durch eine Erwärmung in seiner Haltefähigkeit, z.B. zum Halten einer Zugkraft oder einer Druckkraft oder einer Scherkraft, geschwächt oder durchgeschmolzen ist, also z.B. an einer Stelle zerteilt, gibt dieses das mit ihm gekoppelte Halteelement frei, sodass der Insertions-Energiespeicher den nicht mehr gesicherten mindestens einen Kanülenhalter relativ zum Kanülengehäuse bewegen und so die Insertionsbewegung beginnen kann. Zur Auslösung der Insertion ist es nicht erforderlich, dass das schmelzbare oder erweichbare Element vollständig durchgeschmolzen wird, da ein z.B. auf Zug beanspruchtes Element zum Beispiel in seiner Haltefähigkeit nur soweit geschwächt werden muss, dass eine anliegende Zugkraft nicht mehr gehalten werden kann und sich beispielsweise ein Halteende des schmelzbaren oder erweichbaren Elementes soweit bewegen oder verschieben kann, dass ein Halteelement aus einer Sperrstellung in die Freigabestellung gebracht wird. Dabei kann es möglich sein, dass das erweichbare oder schmelzbare Element zum Beispiel durch Erwärmung aufgeweicht wird und ein Verlängern oder sogar Durchtrennen oder Aufreißen des schmelzbaren Elementes erst durch die Kraft des auf das schmelzbare Element wirkenden Injektions-Energiespeichers bewirkt wird. Ein vollständiges Durchtrennen des aufweichbaren oder schmelzbaren Elementes, welches auch als Schmelzsicherung bezeichnet werden kann, ist jedoch nicht zwingend erforderlich.

Das schmelzbare oder aufweichbare Element oder Schmelzsicherungselement ist z.B. an einer Seite fest gelagert, z.B. fest am oder unbewegbar relativ zum Kanülengehäuse vorgesehen und zum Beispiel an einer Schmelzsicherungs-Halterung befestigt. An dem der Halterung gegenüberliegenden Ende des Schmelzsicherungselementes kann die Schmelzsicherung mit einem Sperr- oder Halteelement des Energiespeichers gekoppelt sein, welches optional selbst von dem Insertions-Energiespeicher mit einer Kraft beaufschlagt werden kann, welche diesen aus der Sperrstellung in eine Freigabestellung drücken kann, sodass diese Kraft z.B. als Zugkraft oder Druckkraft auf das Schmelzsicherungselement wirken kann. Das Sicherungselement kann auch in zwei gegenüberliegende Richtungen belastet werden, beispielsweise von zwei Federarmen.

Gemäß einer weiteren Ausführungsform kann die Schmelzsicherung aus einem elektrisch leitenden (i.e. mit passendem elektrischen Widerstand) und aufweichbaren oder schmelzbaren Material bestehen, welches wie oben für die Schmelzsicherung beschrieben mit dem Sperr- oder Halteelement des Insertions-Energiespeichers gekoppelt und zum Beispiel auf Zug oder alternativ auf Druck belastet ist. Zur Auslösung der Insertion, also zur Freigabe des Sperr- oder Halteelements durch Bewegung der Schmelzsicherung oder eines Teils oder Endes davon, bedingt durch Aufweichung oder Schmelzung, kann eine elektrische Spannung angelegt werden, durch welche ein Strom durch das aufweichbare oder schmelzbare und elektrisch leitende Material, wie zum Beispiel elektrisch leitender Kunststoff oder ein leicht aufweichbares Metall, fließt und hierdurch eine Erwärmung verursacht.

Sofern ein Sicherungselement, welches ein Sperr- oder Halteelement des Insertions-Energiespeichers in Sperrstellung hält, auf Zug beansprucht ist, kann es als flächiges oder stabförmiges Element ausgebildet sein und kann zum Beispiel eine Verjüngung oder Solltrennstelle aufweisen, welche bei Erwärmung einfach und z.B. mit möglichst geringem Energieaufwand aufzuweichen und zum Beispiel zu verlängern oder durchzutrennen ist.

Gemäß einem anderen Aspekt der Erfindung wird das Sperr- oder Halteelement des Insertions-Energiespeichers gesichert, indem ein aufweichbares oder schmelzbares Element verwendet wird, das auf Druck belastet wird. Das Halteelement, welches wie oben beschrieben bedingt durch den Druck (oder alternativ durch eine Zugkraft) des Insertions-Energiespeichers von der Sperrstellung in die Freigabestellung gelangen will, wird in der Sperrstellung durch ein aufweichbares oder schmelzbares Element gehalten oder in die Sperrstellung gedrückt. Das das Sperr- oder Halteelement sichernde Element besteht zumindest teilweise oder vollständig aus einem aufweichbaren oder schmelzbaren Material, wie zum Beispiel Kunststoff oder Metall, welches durch Erwärmung aufgeweicht oder zumindest zum Teil zum Schmelzen gebracht wird. Dabei ist dieses Material zwischen dem auf dieses Material drückenden Sperr- oder Halteelement und einem dem Anlagepunkt zum Halteelement gegenüberliegenden Gegendruckpunkt oder Abstützungspunkt so angeordnet, dass sich das Sicherungsmaterial an dem Abstützungspunkt, welcher beispielsweise fest am oder im oder unbewegbar relativ zum Kanülengehäuse vorgesehen ist, abstützt. Das sichernde Material wird vorzugsweise in einem Bereich zwischen dem Kontakt zum Sperr- oder Halteelement einerseits und zum Abstützungspunkt andererseits so geführt, dass das sichernde Material verschiebbar gelagert ist, also dass es sich zum Beispiel vom Halteelement wegbewegen könnte, wenn der Abstützpunkt nicht vorhanden wäre oder wenn die Länge des sichernden Materials verkürzt würde. Eine Verkürzung der Länge des sichernden Materials kann zum Beispiel dadurch erreicht werden, dass das sichernde Material zumindest teilweise oder vollständig erwärmt wird, zum Beispiel durch ein Erwärmungs- oder Heizelement am Bereich des Abstützpunktes und/oder im Bereich zwischen dem Abstützpunkt und dem Halteelement. Durch eine Erwärmung des sichernden Materials kann dieses an der Erwärmungsstelle, zum Beispiel im Bereich des Abstützpunktes, aufgeweicht werden, sodass es unter der von dem Sperr- oder Halteelement herrührenden Druckbelastung, verursacht z.B. durch die Insertionsfeder, aufweicht oder schmilzt und unter dem anliegenden Druck nachgibt. Bei ausreichender Erwärmung oder Schmelzung gibt das sichernde Material gegen den Druck des Sperr- oder Halteelements so weit nach, dass das Halteelement von einer Sperrstellung in eine Freigabestellung gelangen kann, was zum Beispiel durch den Druck des Insertions-Energiespeichers erfolgen kann. Dabei ist es nicht erforderlich, dass das vollständige sichernde Material erweicht oder geschmolzen wird. Prinzipiell ist es ausreichend, wenn bedingt durch einen Aufweichungs- oder Schmelzvorgang das sichernde Material in seiner Abstützfähigkeit so weit geschwächt wird oder dass dessen Länge so weit reduziert wird, dass das auf das sichernde Material drückende Sperr- oder Halteelement nicht mehr von dem sichernden Material in Sperrstellung gehalten werden kann.

Gemäß einem weiteren Aspekt kann ein sicherndes Material verwendet werden, welches von dem Sperr- oder Halteelement des Insertions-Energiespeichers auf Druck beaufschlagt wird und gegen einen Gegendruckpunkt oder Anlagepunkt drückt, der vorzugsweise gehäusefest ist oder relativ zum Insertionsgehäuse nicht bewegt werden kann. Das sichernde Material kann beispielsweise als Stößel angesehen werden, der an der dem Sperr- oder Halteelement gegenüberliegende Seite auf ein Freigabeelement drückt. Der Stößel kann zwischen den Kontaktpunkten zum Sperr- oder Halteelement und zum Freigabeelement verschiebbar gelagert sein, sodass der Stößel bei einem von dem Sperr- oder Halteelement herrührenden Druck auf das Freigabeelement gedrückt wird und seitlich nicht ausweichen kann. Das Freigabeelement kann als aufweichbares oder schmelzbares Element gebildet sein, zum Beispiel in Gestalt eines durch Wärme aufweichbaren oder schmelzbaren Kunststoffes oder Metalls, und/oder kann als elektrisches Widerstandselement ausgebildet sein, welches die Fähigkeit mechanischen Gegendruck auf den Stößel auszuüben allmählich verliert, wenn dieses erwärmt oder geschmolzen oder von einem Strom durchflossen wird. Wenn zum Beispiel ein flächiger elektrischer Widerstand als Freigabeelement verwendet wird, kann dieser beispielsweise zur Freigabe von einem Strom durchflossen werden und so zum Beispiel aufgeweicht oder ausgelötet oder weggelötet werden, da der hindurchfließende Strom zum Beispiel zur Erwärmung und Schmelzung des Materials oder eines verwendeten Lotes führt, sodass der Stößel keine durch eine Gegendruckstelle erzeugte haltende Kraft gegen die Druckkraft des Sperr- oder Halteelements entgegensetzen kann, wodurch das Sperr- oder Halteelement den Stößel verschiebt und so die Entladung des Insertions-Energiespeichers ermöglicht. Dabei kann der Stößel durch die Stelle hindurchtreten, an welcher sich vor der Auslösung, also z.B. vor dem Weglöten oder Auslöten, das Freigabeelement befunden hatte.

Gemäß einem weiteren Aspekt wird die Sicherung des Sperr- oder Halteelementes des Insertions-Energiespeichers gegen eine Auslenkung mittels eines flächigen Elements oder Folienelements realisiert. Dabei kann das Flächenelement oder die Folie so über dem Sperr- oder Halteelement angebracht werden, dass die Folie die in Freigaberichtung wirkende Kraft des Halteelements halten kann. Beispielsweise kann die Folie so über dem Halteelement und mindestens zwei angrenzenden Bereichen, z.B. auf einer Oberfläche des Kanülengehäuses, aufgebracht oder aufgeklebt oder eingespannt werden, dass das Halteelement nicht in Freigabeposition gelangen kann, solange die Folie vorhanden oder gespannt ist, da die Folie an der Position liegt, an welcher sich das Haltelement in Freigabeposition befinden würde. Die Folie nimmt somit den Druck des Sperr- oder Halteelements auf und hält dieses in Sperrstellung. Beispielsweise kann die Folie an dem Kanülengehäuse oder an dem Kanülenhalter befestigt sein, um das Halteelement in Sperrstellung zu halten. Gemäß einer Variante kann die Folie auch in Form separater einzelner Folienelemente vorgesehen sein, welche zum Beispiel zwei oder mehr Halteelemente halten, indem die Folie oder Folienelemente über den Halteelementen so angebracht oder verklebt oder verspannt sind, dass die Halteelemente nicht in die Freigabeposition gelangen können.

Die nachfolgende Beschreibung erfolgt zur Vereinfachung anhand von Beispielen für ein Halteelement und ein Sicherungselement, wobei in einer konkreten Ausführungsform der Erfindung auch zwei, drei oder mehr Halteelemente vorgesehen sein können, die von einem, zwei, drei oder mehr Sicherungselementen gehalten werden, ohne dass dies nachfolgend nochmals ausdrücklich wiederholt wird.

Zur Freigabe des Sperr- oder Halteelements kann die Folie erwärmt werden, um so zum Beispiel die Haltefähigkeit der Folie soweit zu schwächen, dass diese die z.B. von einer Insertionsfeder verursachte Freigabekraft des Halteelements nicht mehr zurückhalten kann, sodass das Sperrelement beispielsweise durch die Folie hindurchtreten kann oder zu einem Dehnen oder Reißen der Folie führen kann, wodurch das Sperr- oder Halteelement in Freigabeposition gelangen kann. Hierzu können ein oder mehrere Heizelemente an der Folie oder in der Nähe der Folie vorgesehen sein, um die Folie durch Erwärmung mechanisch entsprechend zu schwächen oder aufzuweichen oder zum Reißen zu bringen. Beispielsweise kann die Folie einen Verjüngungsbereich aufweisen, welcher von einem Heizelement erwärmt werden kann, um so zum Beispiel ein Aufweiten oder Reißen der Folie zu erreichen.

Gemäß einem weiteren Aspekt weist ein Insertionsmechanismus für eine Kanüle ein Kanülengehäuse sowie einen relativ dazu bewegbaren Kanülenhalter auf, welcher durch einen Insertions-Energiespeicher mit einer Kraft beaufschlagt ist, welche in eine Richtung wirkt, in der sich der Kanülenhalter relativ zum Kanülengehäuse bewegen kann. Allgemein wird auf obige Ausführungen zur Ausgestaltung eines Insertionsmechanismus und dessen Komponenten verwiesen, wobei bei der vorliegenden Ausführungsform kein Sperr- oder Halteelement zum Halten des Kanülenhalters relativ zum Kanülengehäuse wie oben beschrieben vorgesehen ist. Stattdessen wird der Kanülenhalter mittels eines Klebers oder einer Klebefläche in einer festen Position relativ zum oder am Kanülengehäuse gehalten. Die Freigabe oder Auslösung zur Insertion kann durch ein Heizelement erfolgen, welches am oder bei der Klebestelle oder dem Klebemittel vorgesehen ist, wobei zum Beispiel das Klebemittel durch Erwärmen seine Haltefähigkeit verliert, wodurch der Kanülenhalter freigegeben wird, sodass sich dieser durch die Kraft des Injektions-Energiespeichers relativ zum Kanülengehäuse verschieben kann. Alternativ oder zusätzlich kann ein Klebemittel verwendet werden, welches leitfähig ist, sodass das leitfähige Klebemittel zur Freigabe mit einem elektrischen Strom beaufschlagt werden kann, welcher durch das Klebemittel fließt und aufgrund des dem Klebemittel innewohnenden Innenwiderstandes zu einer Erwärmung des Klebemittels und somit zur Reduktion der Klebekraft und folglich zur Freigabe des Kanülenhalters im Kanülengehäuse führt.

Gemäß einem weiteren Aspekt betrifft die Erfindung eine Verabreichungsvorrichtung zur Verabreichung einer Substanz mit einem Insertionsmechanismus wie vorhergehend beschriebenen und mit einem Reservoir für die Substanz und/oder einer Pumpe, welche mit dem proximalen Ende einer Kanüle verbunden ist. Die Kanüle kann an dieser Verbindungsstelle, wie z.B. oben beschrieben, eine Hartkanüle und/oder Softkanüle sein und kann auch mittels eines vergleichsweise weichen Schlauches mit dem Reservoir verbunden sein. Hierdurch können z.B. Verlustkräfte, welche beim Verbiegen der Stahlkanüle entstehen können, reduziert werden.

Gemäß einem weiteren Aspekt betrifft die Erfindung ein Verfahren zum automatischen Ausstoßen oder Vorschieben einer Kanüle unter Verwendung eines wie oben beschriebenen Insertionsmechanismus. Das Verfahren kann durchgeführt werden ohne dass die vorgeschobene oder zum Beispiel aus dem Kanülengehäuse herausgeschobene Kanüle in ein Gewebe einsticht und bezieht sich allgemein nur auf den Auslösevorgang oder Freigabevorgang der Kanüle vor einer optionalen späteren Insertion oder Nicht-Insertion. Das Verfahren umfasst die Schritte des Erwärmens einer Sicherungsstruktur, wodurch diese zum Beispiel geschwächt wird oder zum Schmelzen oder Verformen oder allgemein zum Freigeben einer Sicherungsposition gebracht wird, wodurch ein Halteelement zum Halten eines Kanülenhalters gegen die Kraft eines Insertions-Energiespeichers freigegeben werden kann.

Allgemein betrachtet betrifft die Erfindung die Freigabe oder Auslösung eines Insertionsmechanismus gemäß einem Ausführungsbeispiel durch Erweichung, welche z.B. durch Erwärmung eines Materials erfolgen kann. Hierzu kann z.B. eine Sicherung oder ein Sicherungselement verwendet werden, welches auf Druck belastet ist oder bei welchem eine Scherkraft anliegt oder welches auf Zug belastet ist, wobei Kombinationen dieser Belastungen anliegen können, wie z.B. Druck und Scherung.

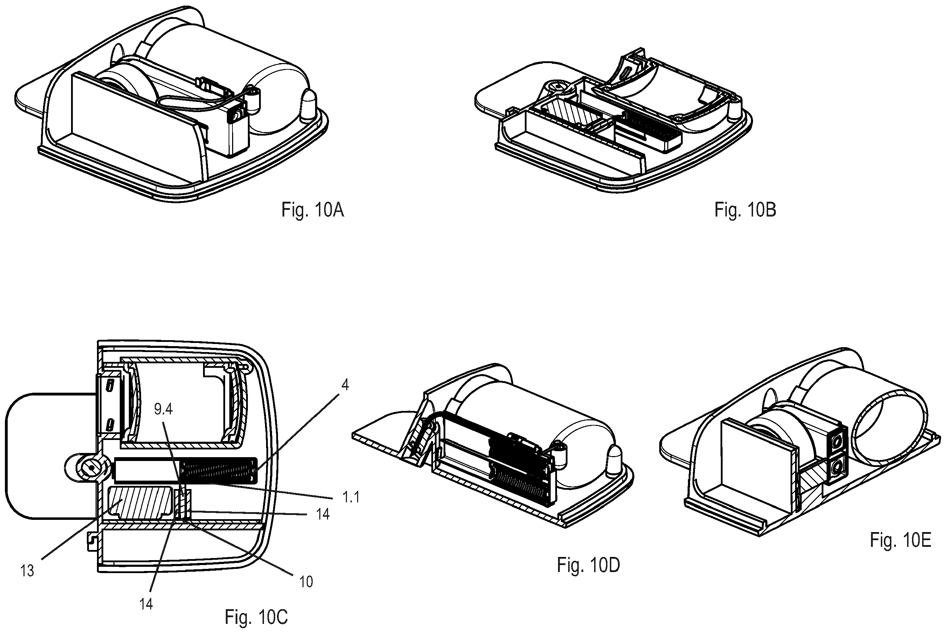

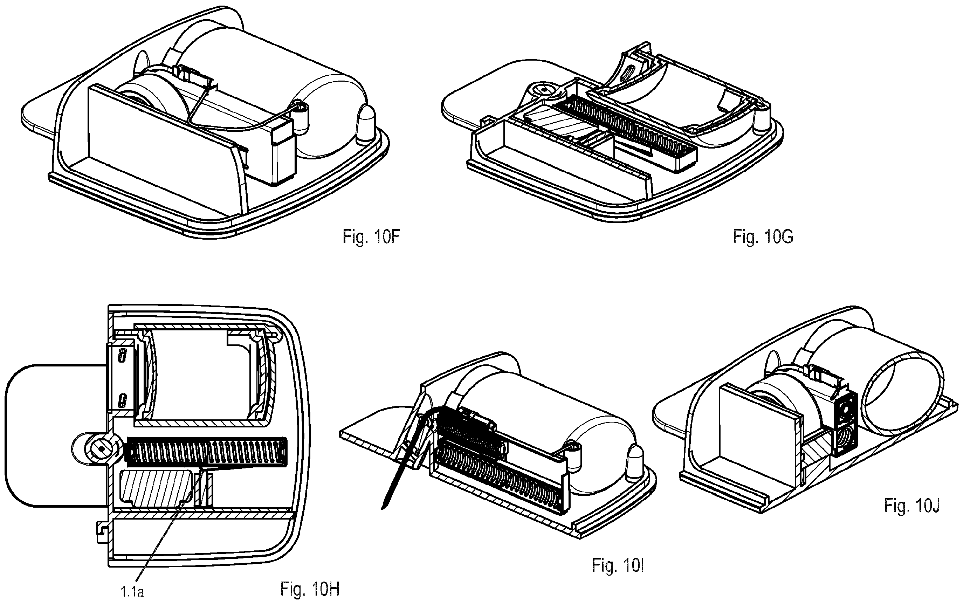

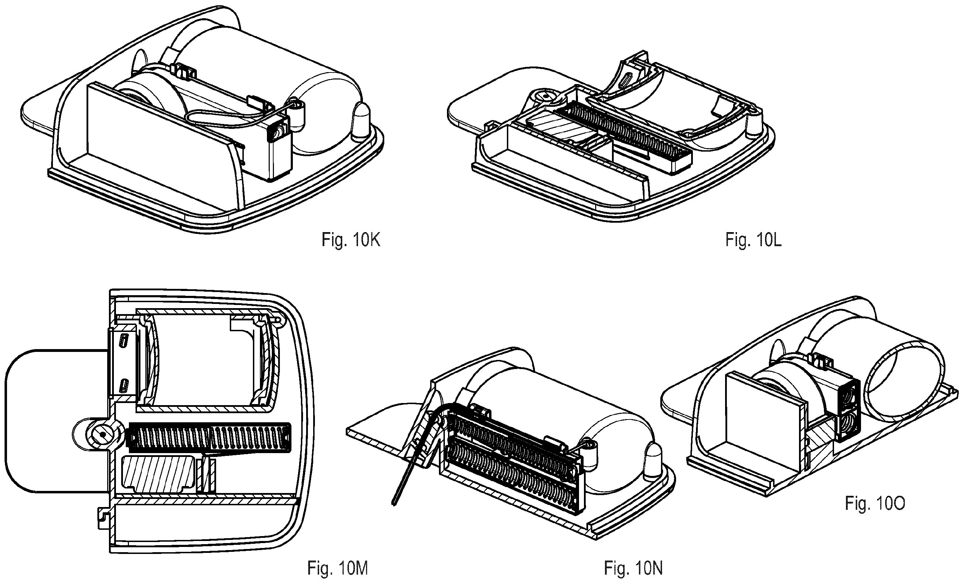

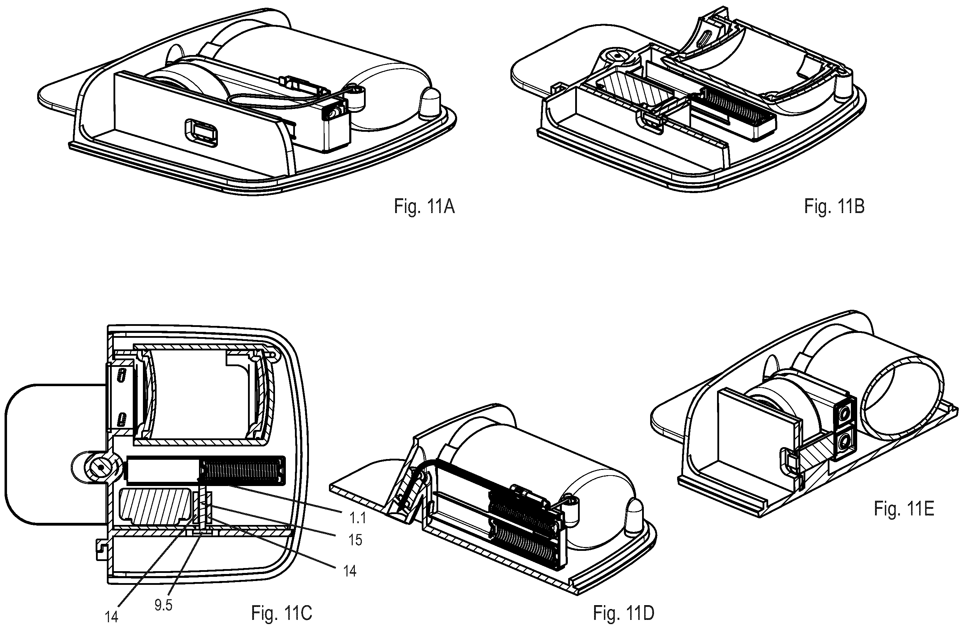

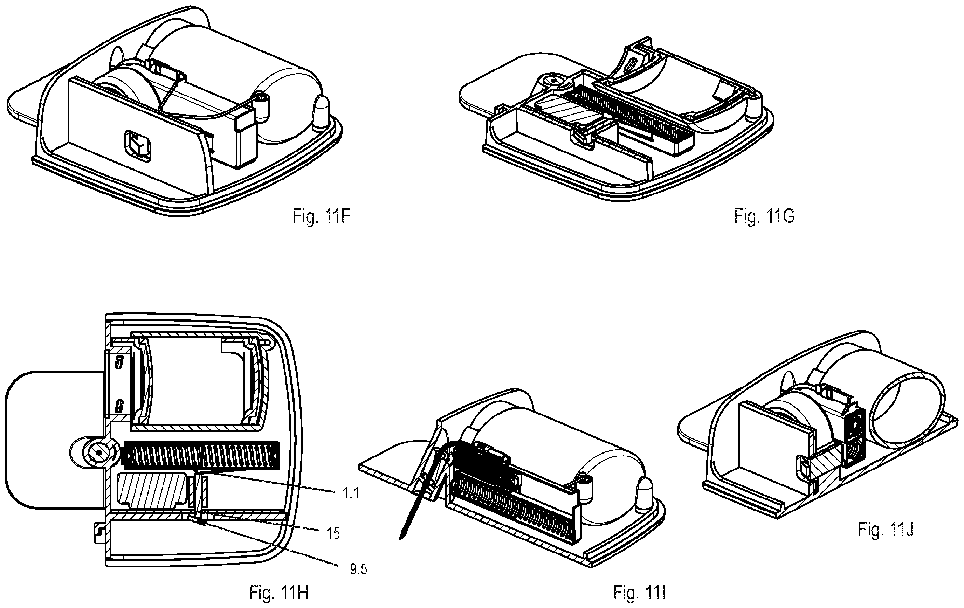

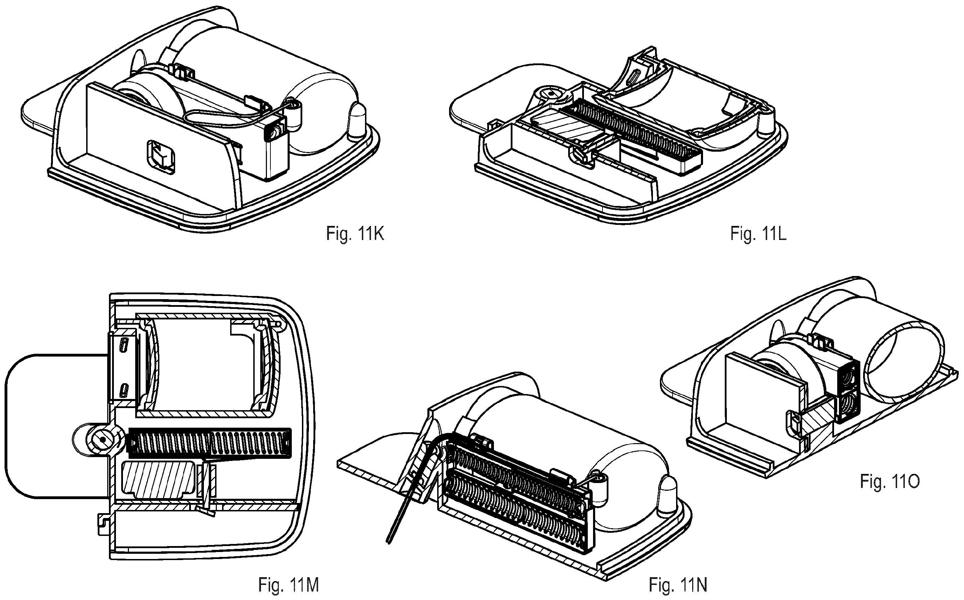

Gemäß einer Ausführungsform kann ein mit Druck belastetes Medium, wie beispielsweise in den nachfolgenden Ausführungsbeispielen gemäß Figur 10 und 11 gezeigt, mittels Wärmezufuhr erweicht oder aufgeschmolzen werden, sodass es einer (Halte-)Belastung nicht mehr standhält und wegweicht. Dieser Mechanismus kann genutzt werden, um einen weiteren Mechanismus, wie beispielsweise das Halten eines Auslöseelements oder RückHalteelements, z.B. irreversibel von einem gesperrten in einen freigebenden Zustand umzuschalten.

Ausführungsformen, bei welchen z.B. ein Sicherungselement auf Druck belastet wird, weisen den Vorteil auf, dass ein Querschnitt während einer längeren z.B. mehrjährigen Lagerung nicht kritisch belastet wird.

Bei einem auf Zug belasteten Sicherungselement muss weniger Material oder weniger Volumen erhitzt werden, da die Belastung im Material immer größer wird, je kleiner der Querschnitt wird, was ein sicheres Lösen oder Öffnen des Sicherungselements vereinfacht.

Die Erfindung wird nachfolgend anhand von Ausführungsbeispielen beschrieben. Es zeigen:

| | |
|---|---|
| Figur 1A | eine Schnittbildansicht eines Zwei-Feder-Insertionsmechanismus im Ausgangszustand; |
| Figur 1B | den in Figur 1A gezeigten Insertionsmechanismus von oben betrachtet; |
| Figur 2 | eine Schnittbildansicht des Insertionsmechanismus mit ausgefahrener Soft- und Stahlkanüle; |
| Figur 3 | den Insertionsmechanismus mit ausgefahrener Softkanüle und zurückgezogener Stahlkanüle; |
| Figur 4 | eine Explosionsansicht der Komponenten des Insertionsmechanismus; |
| Figur 5 | eine Perspektivansicht des Insertionsmechanismus mit Sperr- bzw. Auslöseelement; |
| Figur 6 | eine Draufsicht auf den Insertionsmechanismus zur Illustration des Sperrelements; |
| Figur 7A und 7B | Schnittansichten der Insertionsvorrichtung beim Lösen der Kopplung von Soft-Kanülenhalter und Hart-Kanülenhalter vor dem Rückzug der Stahlkanüle; |
| Figur 8 | eine erste Ausführungsform einer automatisch lösbaren Sicherung; |
| Figur 9 | eine zweite Ausführungsform einer automatisch lösbaren Sicherung; |
| Figur 10 | eine dritte Ausfuhrungsform einer automatisch lösbaren Sicherung; |
| Figur 11 | eine vierte Ausführungsform einer automatisch lösbaren Sicherung; |
| Figur 12 | eine fünfte Ausführungsform einer automatisch lösbaren Sicherung; |
| Figur 13 | eine sechste Ausführungsform einer automatisch lösbaren Sicherung; |
| Figuren 14 bis 18 | zeigen Ausführungsformen mit auf Druck belastetem S icherungselement; |

Figuren 19 und 20     zeigen Ausführungsformen mit einer kombinierten Druck- und Scherbelastung;

Figuren 21 und 22     zeigen Ausführungsformen mit kombinierten Sperr- und Heizelementen;

Figuren 23 bis 25     zeigen Ausführungsformen mit auf Zug belasteten Sicherungselementen;

Figuren 26 und 27     zeigen Ausführungsformen, bei welchen ein Sicherungselement ausgelötet wird.

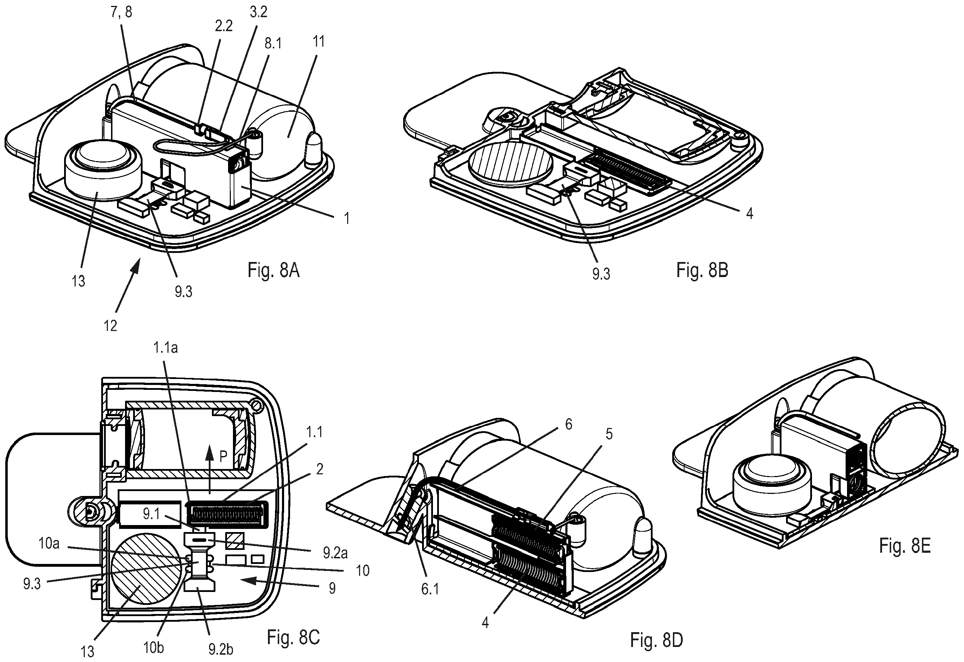

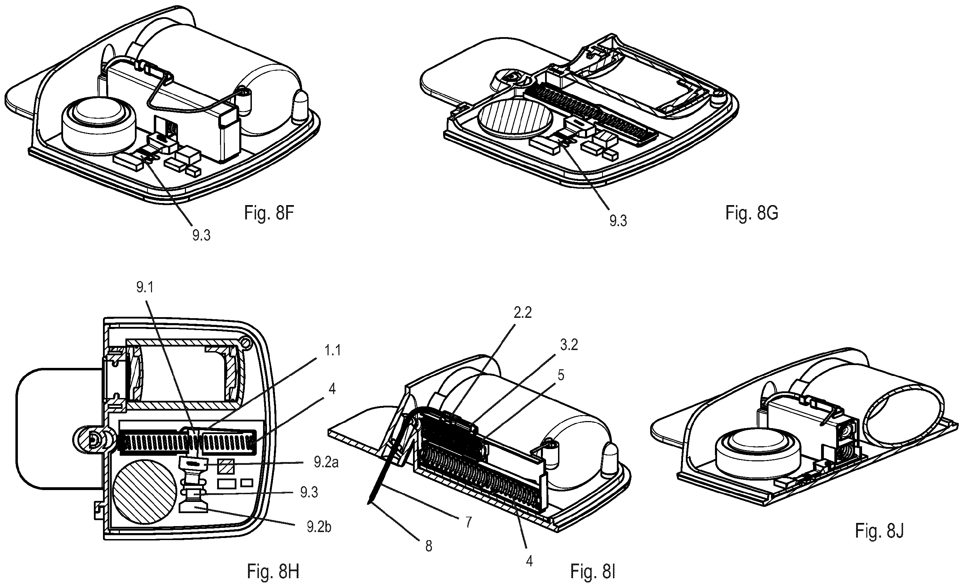

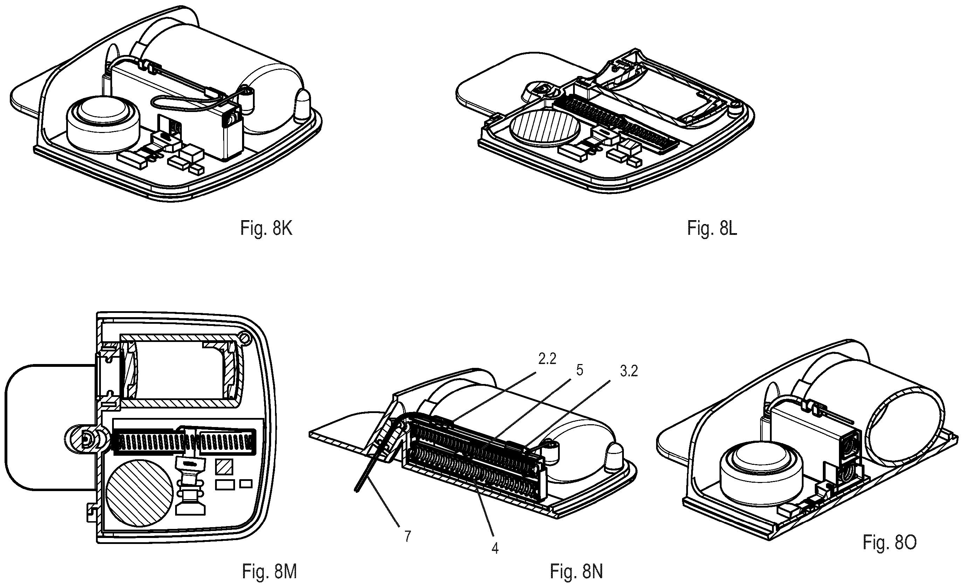

In den Figuren 8 bis 13 bezeichnen die Teilfiguren A bis E Ansichten der jeweiligen Ausführungsform im Ausgangszustand mit intaktem Sicherungselement, die Figuren F bis J zeigen den Zustand nach Lösen des Sicherungselementes und nachfolgendem Ausschub der Kanüle und die Teilfiguren K bis O zeigen Ansichten des Insertionsmechanismus mit ausgeschobener Softkanüle und zurückgezogener Hartkanüle.

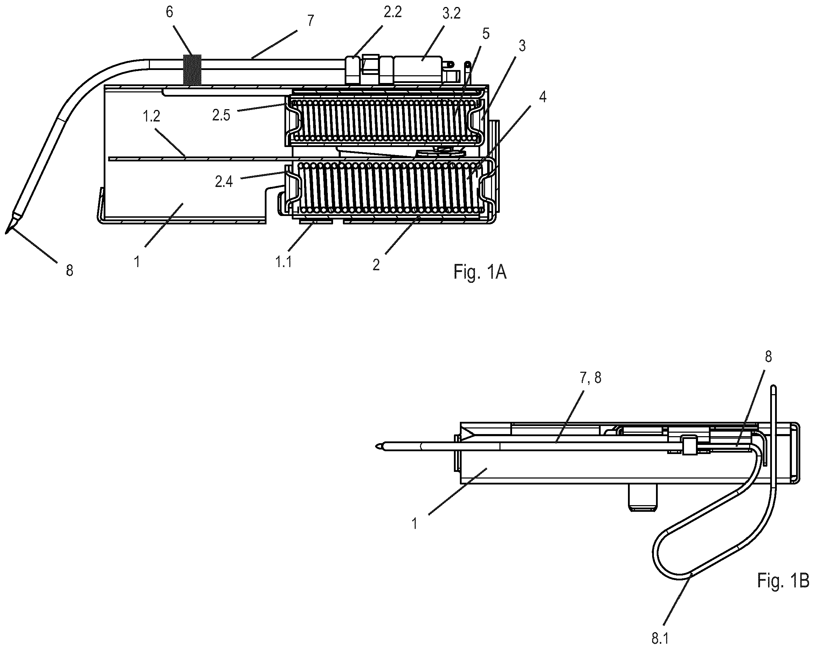

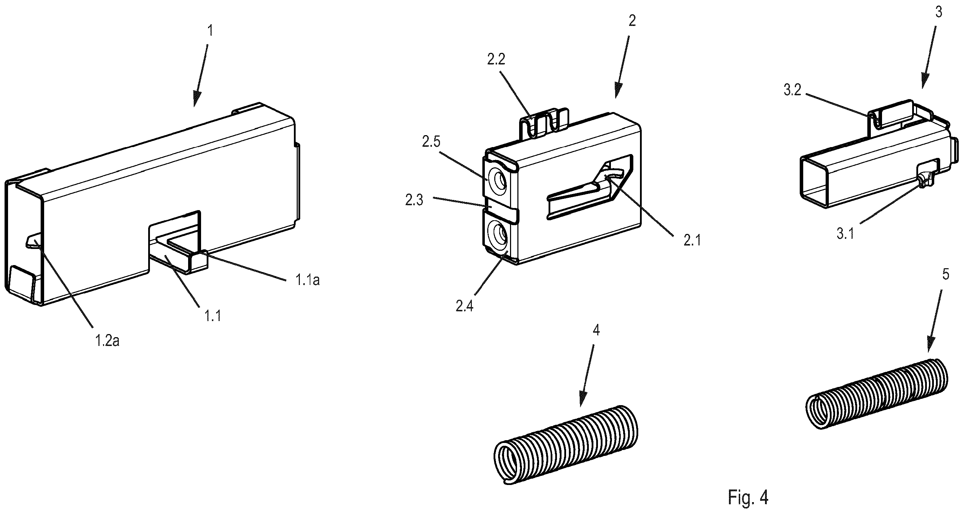

Figur 1A zeigt einen Zwei-Federinsertionsmechanismus in Schnittansicht mit wegabhängiger Ablaufsteuerung. Der Insertionsmechanismus ist in der gezeigten Ausführungsform als Kanülen-Einstech- und Rückzugsmechanismus ausgebildet und kann nach Auslösung automatisch eine Hart- oder Stahlkanüle 8 zusammen mit der diese Stahlkanüle 8 umgebenden Softkanüle 7 ausschieben und nach erfolgtem Ausschubvorgang die Hartkanüle 8 innerhalb der Softkanüle 7 zurückziehen, wobei die ausgefahrene Softkanüle 7 im ausgefahrenen Zustand verbleibt. Falls die Kanüle 7, 8 vor dem Ausschieben auf einen Körper aufgebracht wird, kann so beispielsweise ein automatischer Insertions- oder Einstech-Vorgang realisiert werden. Die Softkanüle 7 ist an einer Halterung oder Führung 2.2 eines in der Explosionsansicht von Figur 4 gezeigten Soft-Kanülenhalters 2 unverschiebbar befestigt oder geführt, welcher relativ zum Kanülengehäuse 1 verschiebbar ist und kann zusammen mit einer Relativbewegung des Soft-Kanülenhalters 2 relativ zum Kanülengehäuse 1 verschoben werden. Die Hartkanüle 8 ist an einer Hartkanülen-Halterung oder Hartkanülen-Führung 3.2 befestigt oder geführt und bewegt sich somit zusammen mit dem Hart-Kanülenhalter 3.

Der Soft-Kanülenhalter 2 ist innerhalb des Kanülengehäuses 1 in Längsrichtung entlang des ebenfalls im Inneren des Kanülengehäuses 1 vorgesehenen Kanülengehäusefingers 1.2, der auch als Führung und Ablaufsteuerung dient, bewegbar. Der Soft-Kanülenhalter 2 weist an seiner in den Figuren 1 bis 7 links gezeigten Stirnseite, welche in Insertions-Richtung des Soft-Kanülenhalters 2 weist, einen Anschlag oder eine Anlagefläche 2.4 für eine Insertionsfeder 4 und eine Anlagefläche oder einen Anschlag 2.5 für den Hart-Kanülenhalter 3 auf, welche durch eine Öffnung 2.3 zum Hindurchtreten der Ablaufsteuerung 1.2 voneinander beabstandet sind. Am Soft-Kanülenhalter 2 ist eine Halterung oder Führung 2.2 für die Softkanüle 7 vorgesehen, welche fest und unverschiebbar mit dem Soft-Kanülenhalter 2 verbunden ist und die Softkanüle 7 vorzugsweise fest und unverschiebbar hält, sodass bei einer Relativbewegung oder Vescie-bebewegung des Soft-Kanülenhalters 2 relativ zum Kanülengehäuse 1 die von dem Soft-Kanülenhalter 2 mitgenommene Softkanüle 7 ebenfalls relativ zum Kanülengehäuse 1 verschoben wird. Beispielsweise kann die Softkanüle 7 mittels Klemmelementen an der Halterung 2.2 befestigt werden und/oder durch ein Klebemittel an der Halterung 2.2 befestigt werden. Die Halterung 2.2 sollte so ausgebildet sein, dass die Softkanüle 7 bei einer Bewegung der Halterung 2.2 bzw. einer Bewegung des Soft-Kanülenhalters 2 mitgenommen werden kann, wobei die Softkanüle 7 so in der Halterung 2.2 vorgesehen oder befestigt ist, dass im Haltezustand eine Substanz durch das Innere der Softkanüle 7 hindurchtreten kann. Im Ausgangszustand ist innerhalb der Softkanüle 7 über deren gesamte Länge und an den Enden überstehend eine Hartkanüle 8 vorgesehen, welche innerhalb der Softkanüle 7 in deren Längsrichtung verschiebbar ist und während eines Ausschubvorgangs oder Insertionsvorgangs die Softkanüle 7 mitnehmen kann oder sich zusammen mit der Softkanüle 7 bewegt.

Die Soft-Kanülenhalterung 2 weist ein Koppel- oder Auslöseelement 2.1 auf, welches im Ausgangszustand eine Kopplung zwischen dem Soft-Kanülenhalter 2 und dem Hart-Kanülenhalter 3 bewirkt, sodass Soft-Kanülenhalter 2 und Hart-Kanülenhalter 3 relativ zueinander nicht oder nur geringfügig verschoben werden können. Eine Bewegung, welche der Soft-Kanülenhalter 2 ausführt, wie zum Beispiel eine Insertionsbewegung, wird von dem Soft-Kanülenhalter 2 mittels des Auslöseelementes 2.1, das zunächst als Koppelelement dient, auf den Hart-Kanülenhalter 3 übertragen. Wird der Soft-Kanülenhalter 2 in Insertionsrichtung (in den Figuren nach links) bewegt, so wird diese Bewegung durch das Koppelelement 2.1 auf den Hart-Kanülenhalter 3 übertragen, wo das Auslöse- oder Koppelelement 2.1 in ein Gegenkoppelelement 3.1 oder einen Hintergriff eingreift. Das Koppelelement 2.1 ist in der Einzelansicht des Soft-Kanülenhalters 2 in Figur 4 in einem nach innen geklappten und entkoppelten Zustand gezeigt. Wird der Soft-Kanülenhalter 2 in das Kanülengehäuse 1 so eingesetzt, dass die Ablaufsteuerung bzw. der Insertionsgehäuse-Finger 1.2 durch die Öffnung 2.3 des Soft-Kanülenhalters 2 hindurchtritt, also zum Beispiel in Figur 4 in der gezeigten Ausrichtung von links stirnseitig in das Kanülengehäuse 1 eingeschoben, so ist das Auslöse- oder Koppelelement 2.1 im vorderen in Figur 4 links gezeigten schmalen Bereich 1.2a der Ablaufsteuerung 1.2 nach innen geklappt, da die Ablaufsteuerung 1.2 im vorderen Bereich schmäler oder verjüngt ist. Im hinteren breiteren Bereich 1.2b wird das Auslöseelement 2.1 durch die in diesem Bereich aufgeweitete oder breitere Ablaufsteuerung 1.2 nach außen gedrückt und ist somit mit dem Gegenkoppelement oder Hintergriff 3.1 des Hart-Kanülenhalters 3 gekoppelt, wodurch Soft-Kanülenhalter 2 und Hart-Kanülenhalter 3 relativ zueinander unverschiebbar sind, solange sich das Auslöseelement 2.1 in dem Bereich 1.2b der Ablaufsteuerung 1.2 befindet, welcher aufgeweitet ist und das Auslöseelement 2.1 in den Eingriff mit dem Gegenkoppelelement 3.1 drückt. Im verjüngten oder schmaleren Bereich 1.2a der Ablaufsteuerung 1.2 kann das Auslöseelement 2.1 nach innen ausweichen und somit vom Gegenkoppelelement 3.1 oder Hintergriff entkoppeln, wodurch die Kopplung zwischen Soft-Kanülenhalter 2 und Hart-Kanülenhalter 3 gelöst wird und beide relativ zueinander bewegbar oder unabhängig voneinander verschiebbar sind.

Der Hart-Kanülenhalter 3 weist eine Halterung oder Führung 3.2 für die Hartkanüle 8 auf, welche im in Figur 1 gezeigten Ausgangszustand vollständig durch die Softkanüle 7 hindurchtritt und von dieser umgeben wird, sodass die Hartkanüle 8 sowohl am distalen Ende aus der Softkanüle 7 heraussteht und am distalen Ende vorzugsweise eine Abschrägung oder Zuspitzung aufweist und am gegenüberliegenden proximalen Ende ebenfalls aus der Softkanüle 7 herausragt und fest und unverschiebbar an der Halterung 3.2 des Hart-Kanülenhalters 3 befestigt ist.

Figur 1B zeigt eine Draufsicht auf den in Figur 1A gezeigten Insertionsmechanismus, aus welchem ersichtlich ist, dass das proximale Ende der Hartkanüle 8 mit einem gebogen eingezeichneten Kanülenelement 8.1 verbunden ist oder sich als dieses Kanülenelement fortsetzt, welches zum Beispiel mit einem Reservoir der abgegebenen Substanz und/oder einem Abgabemechanismus, wie zum Beispiel einer Pumpe, verbunden sein kann. Die in Figur 1B gezeigte schleifenförmige Struktur 8.1 des Kanülenelements kann sicherstellen, dass die zum Beispiel in Figur 1B rechts gezeigte Seite der schleifenförmigen Struktur 8.1 ortsfest relativ zum Kanülengehäuse 1 verbleiben kann und z.B. daran befestigt ist und die Hartkanüle 8 zumindest in deren distale Richtung verschoben werden kann, ohne dass zum Beispiel eine Zugkraft an der Befestigungsstelle des Kanülenabschnittes 8.1 relativ zum oder am Kanülengehäuse 1 (proximaler Bereich des Kanülenabschnittes 8.1) auftritt. Im gegenüberliegenden distalen Bereich des Kanülenabschnitts 8.1 kann sich dieser zusammen mit der Hartkanüle 8 bewegen und bei einer in Figur 1B nach links gerichteten Bewegung der Kanüle 8 in Insertionsrichtung ergibt sich eine Aufweitung der gezeigten Schleifen- oder Bogenform des Kanülenabschnitts 8.1.

Im in Figur 1A gezeigten Ausgangszustand ist eine Insertionsfeder 4, welche als Druckfeder ausgebildet ist, am proximalen Ende des Insertionsmechanismus an dem Kanülengehäuse 1 abgestützt und am gegenüberliegenden Ende an der Anlagefläche 2.4 des Soft-Kanülenhalters 2. Der Soft-Kanülenhalter 2 wird durch die Insertionsfeder 4 somit relativ zum Kanülengehäuse 1 in Ausschubrichtung oder Insertionsrichtung der Kanüle gedrückt und von einem Sperr- oder Auslöseelement 1.1, welches ein beweglicher oder auslenkbarer Bestandteil des Kanülengehäuses 1 ist, in Position gehalten. Dabei kann gemäß einer Ausführungsform das Sperr- oder Auslöseelement 1.1 so ausgestaltet sein, dass dieses von dem von der Insertionsfeder 4 erzeugten Druck aus der Sperrstellung in eine Freigabestellung gedrückt wird oder würde, wenn das Auslöseelement 1.1 nicht durch eine später beschriebene Sicherung oder ein Sicherungselement in der gezeigten Sperrstellung gehalten wird. Beispielsweise kann das Sperr- oder Auslöselement 1.1 so ausgebildet sein, dass dieses unmittelbar von der Insertionsfeder 4 oder von dem Soft-Kanülenhalter 2 oder optional auch von dem Hart-Kanülenhalter 3 in Sperrstellung zum Beispiel über eine Anschrägung 1.1a oder Funktionsfläche so mit einer Kraft beaufschlagt wird, dass das Auslöseelement 1.1 in Richtung einer Freigabestellung mit einer Kraft beaufschlagt wird.

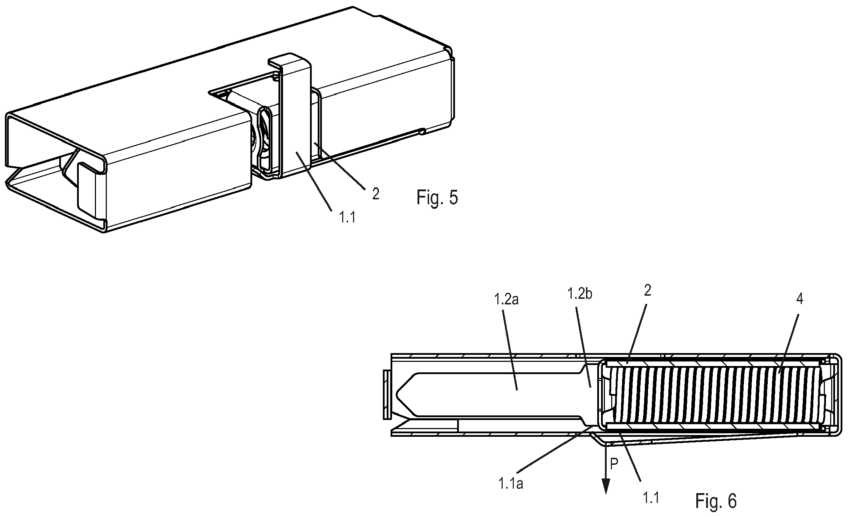

Im gezeigten Ausführungsbeispiel greift das Auslöseelement 1.1 des Kanülengehäuses 1 in einen Eingriff oder vor eine Stirnseite des Soft-Kanülenhalters 2 ein und hält den Soft-Kanülenhalter 2 gegen die Spannung der Insertionsfeder 4 in der hinteren proximalen Position, wie beispielsweise in den Figuren 5 und 6 gezeigt. Die im Ausführungsbeispiel der Figur 6 gezeigte Abschrägung oder Funktionsfläche 1.1a des Auslöseelementes 1.1 erfährt in Druckrichtung der Insertionsfeder 4 eine Kraft, welche bewirkt, dass das Auslöseelement 1.1 in die durch den Pfeil P angezeigte Richtung, also beispielsweise in Richtung auf die Außenseite des Kanülengehäuses 1, quer oder senkrecht zur Wirkrichtung der Insertionsfeder 4 gedrückt wird, um so die Haltekraft des Soft-Kanülenhalters 2 durch das Auslöseelement 1.1 zu lösen und somit den Soft-Kanülenhalter 2 freizugeben, sodass der Soft-Kanülenhalter 2, angetrieben durch die Kraft der Insertionsfeder 4, welche sich gegen das Kanülengehäuse 1 abstützt, in Insertionsrichtung bewegt oder geschoben werden kann, nachdem das Auslöseelement 1.1 freigegeben wurde, indem zum Beispiel eine Sicherung, welche das Auslöseelement 1.1 in der Sperrstellung hält, gelöst oder geöffnet wurde.

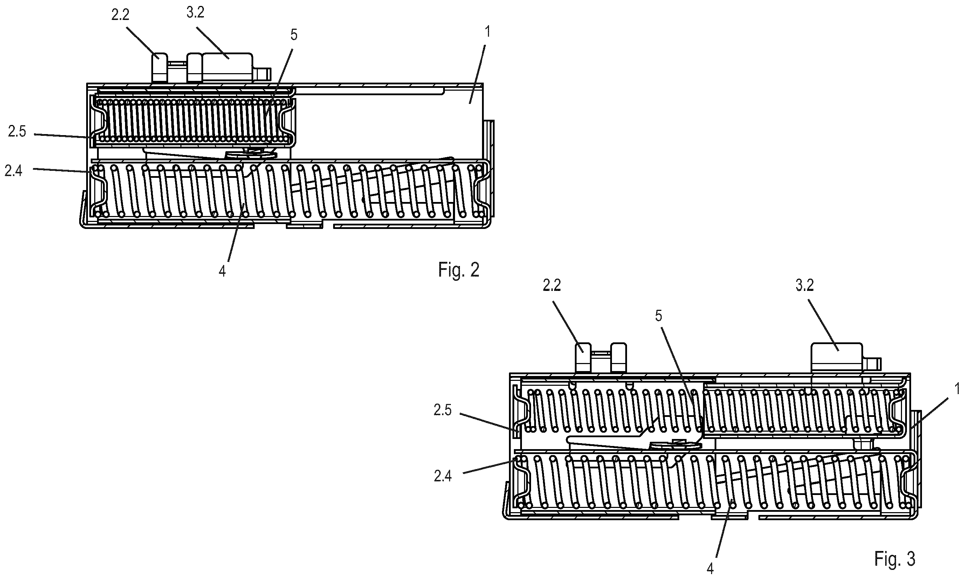

Nach dem Freigeben des Soft-Kanülenhalters 2 drückt die Insertionsfeder 4 den Soft-Kanülenhalter 2 in distale Richtung des Kanülengehäuses 1, wie in Figur 2 gezeigt, bis der Soft-Kanülenhalter 2 innerhalb des Kanülengehäuses 1 an eine vordere Anschlagsposition des Kanülengehäuses 1 gelangt. Während dieser Vorschub- oder Insertionsbewegung nimmt der Soft-Kanülenhalter 2 durch die Kopplung des Auslöseelements 2.1 mit dem Gegenkoppelement 3.1 des Hart-Kanülenhalters 3 den Hart-Kanülenhalter 3 mit und verschiebt diesen ebenfalls in distale Richtung relativ zum Kanülengehäuse 1. Während dieser gemeinsamen Bewegung von Softkanülenhaler 2 und Hart-Kanülenhalter 3 werden auch die Soft-Kanülenhalterung 2.2 und die Hart-Kanülenhalterung 3.2 relativ zum Kanülengehäuse 1 in distale Richtung verschoben, wie ebenfalls in Figur 2 gezeigt, wodurch die Soft- und Hartkanüle 7 und 8 gemeinsam in distale Richtung geschoben werden. Im Falle des Anbringens des Kanülengehäuses 1 und insbesondere des Aufbringens der distalen Spitze der Hartkanüle 8 auf einer Hautoberfläche kann so ein Einstich- oder Insertionsvorgang erfolgen. Selbstverständlich kann dieser Funktionsablauf auch durchgeführt werden, ohne dass eine Insertion erfolgen muss, beispielsweise um die Funktionsfähigkeit der Insertionsvorrichtung zu überprüfen. Figur 2 zeigt den Zustand des Insertionsmechanismus bei ausgeschobener und beispielsweise eingestochener Soft- und Hartkanüle 7, 8.

Die Rückzugsfeder 5 stützt sich einerseits gegen eine Anlage- oder Anschlagsfläche 2.5 des Soft-Kanülenhalters 2 ab und ist im Ausführungsbeispiel als gespannte Druckfeder ausgebildet, welche am gegenüberliegenden Abstützpunkt auf eine Anlage- oder Anschlagsfläche des Hart-Kanülenhalters 3 drückt oder an dieser anliegt.

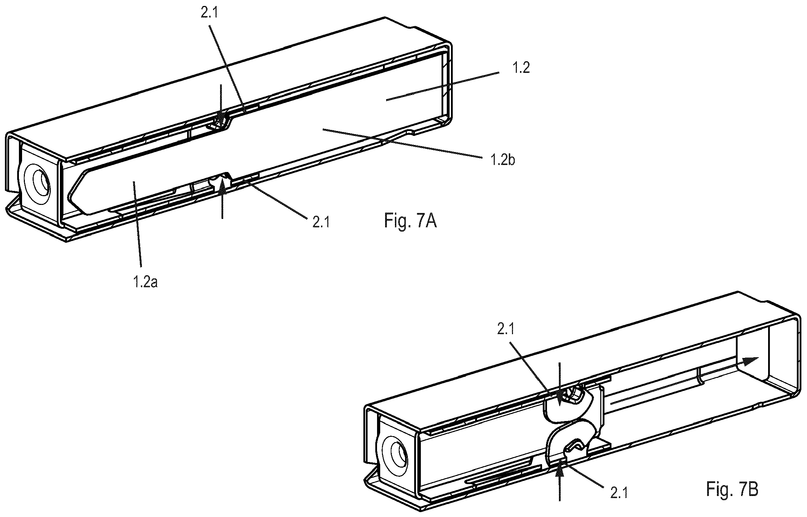

Im in Figur 2 gezeigten Zustand ist das Auslöseelement oder Koppelelement 2.1 des Soft-Kanülenhalters 2, welches in den Hintergriff 3.1 oder das Gegenkoppelement des Hart-Kanülenhalters 3 eingreift, aus der gekoppelten Stellung, in welcher dieses Auslöseelement 2.1 gehalten oder gedrückt wird, bedingt durch den breiteren Bereich 1.2b der Ablaufsteuerung oder Führung 1.2 des Kanülengehäuses 1, gelöst, da sich das Gegenkoppel- oder Auslöseelement 3.1 in dem distalen Bereich des Kanülengehäuses 1 befindet, wo die Ablaufsteuerung 1.2 verjüngt oder schmäler ist, sodass das Auslöseelement 2.1 im gezeigten Ausführungsbeispiel in Richtung auf das Gehäuseinnere klappen kann (siehe Figuren 7A und 7B) und so eine Entkopplung zur Gegenkoppelstelle oder zum Hintergriff 3.1 des Hart-Kanülenhalters 3 ermöglicht. Hierdurch wird der Hart-Kanülenhalter 3 vom Soft-Kanülenhalter 2 entkoppelt und kann sich relativ zu diesem bewegen.

Hierdurch kann sich die Rückzugsfeder 5 entspannen und drückt, wie in Figur 3 gezeigt, den Hart-Kanülenhalter 3 wieder zurück in proximale Richtung, bis dieser an einer Anschlagsfläche des Kanülengehäuses 1 anliegt. Hierdurch wird auch die Hart-Kanülenhalterung 3.2 relativ zur Soft-Kanülenhalterung 2.2 verschoben, wodurch die Hartkanüle 8 relativ zur Softkanüle 7 verschoben und innerhalb dieser zurückgezogen wird. Im Falle einer optionalen Insertion würde so zum Beispiel die Softkanüle 7 eingestochen bleiben, während die Hartkanüle 8 aus der Einstichstelle zurückgezogen wird.

Das Auskoppeln zwischen Soft-Kanülenhalter 2 und Hart-Kanülenhalter 3 findet dann statt, wenn im Laufe der nach distal gerichteten Insertionsbewegung des Soft-Kanülenhalters 2 das Auslöseelement 2.1 in den Bereich der Umschaltgeometrie oder Verjüngung der Ablaufsteuerung 1.2, also den Übergang von 1.2b nach 1.2a, gelangt, wodurch das Auslöseelement 2.1 zum Beispiel bedingt durch die Kraft der Rückzugsfeder 5 aus der Halteposition in dem Gegenkoppelelement 3.1 des Hart-Kanülenhalters 3 in eine Freigabestellung gedrückt oder gezogen wird.

Die Softkanüle 7 ist koaxial und dichtend über der Hart- oder Stahlkanüle 8 angeordnet. Die zum Beispiel geschliffene Spitze der Stahlkanüle oder Hartkanüle 8 ragt zum Beispiel um wenige Millimeter aus der Softkanüle 7 heraus, um ein möglichst reibungsloses oder schmerzfreies Einstechen in die Haut zu ermöglichen. Zum Einstechen werden Hart- oder Stahlkanüle 8 und Softkanüle 7 gleichzeitig zur Injektionsstelle hin bewegt. Nach dem Erreichen der Injektionstiefe, was zum Beispiel im in Figur 2 gezeigten Zustand des Insertionsmechanismus der Fall sein kann, wird die Softkanüle 7 in der vorderen oder distalen Position z.B. durch eine Schnappverbindung oder Haltevorrichtung, wie z.B. einen oder mehrere Schnapper oder Haken, oder durch eine Feder, fixiert oder zum Beispiel durch die Kraft der entspannten Insertionsfeder 4 in der vorderen Position gehalten. Anschließend kann die Hart- oder Stahlkanüle 8 aus der Insertionsstelle herausgezogen oder relativ zur Softkanüle 7 zurückgezogen werden. Es verbleibt eine koaxiale und dichtende Überlappung zwischen Hart- und Softkanüle 8 und 7. Die optional nachfolgende Verabreichung eines Fluids erfolgt von einem Reservoir und/oder einer Pumpe über die Hartkanüle 8 zur Softkanüle 7 in die Injektionsstelle.

Die Softkanüle 7 wird durch eine Kanülenführung 6, welche zum Beispiel gehäusefest am Kanülengehäuse 1 unverschiebbar zum Kanülengehäuse 1 angebracht ist, geführt, wobei die Softkanüle 7 relativ zur Kanülenführung 6 durch diese geschoben werden kann. Die Kanülenführung 6 kann so angeordnet sein, dass sich der Abstand zum Auslöseelement 2.1 des Soft-Kanülenhalters 2 bei Betätigung verkleinert.

Optional können ergänzend zum oben beschriebenen Aulöseelement 2.1 noch ein oder mehrere Auslöseelemente 2.1 vorgesehen sein, welche ebenfalls die oben beschriebene Funktionalität aufweisen, um das Sichern oder Sperren des Soft-Kanülenhalters 2 gegen die Kraft der Feder 4 in Ausgangsposition sicherer oder zuverlässiger auszugestalten.

Die verbleibende Spannung der Insertionsfeder 4 und der Rückzugsfeder 5 bewirken, dass der Soft-Kanülenhalter 2 und der Hart-Kanülenhalter 3 in ihren jeweiligen Endpositionen (siehe Figur 3) gehalten werden.

Obwohl die Erfindung anhand des obigen Ausführungsbeispiels unter Verwendung eines Zwei-Feder-Mechanismus beschrieben wird, kann diese prinzipiell auch mit einem Ein-Federmechanismus verwendet werden. Ein Ein-Feder-Injektionsmechanismus ist beispielsweise in den Schweizer Patentanmeldungen 00061/17, 00063/17, 00062/17 und 00208/17 beschrieben, deren Offenbarungsgehalt hiermit in diese Anmeldung aufgenommen wird. Dabei kann beispielsweise angetrieben durch eine einzige Feder eine Soft- und Stahlkanüle aus einer Insertionsvorrichtung ausgeschoben und beispielsweise eingestochen werden, wobei nach erfolgter Insertion die Feder nicht vollständig entspannt ist und ein Antriebsmechanismus umgekoppelt werden kann, sodass mit der verbleibenden Feder-Energie beispielsweise die Hartkanüle aus der Softkanüle herausgezogen werden kann.

Anstelle des Zwei-Feder-Mechanismus können auch beispielsweise vier Federn vorgesehen sein, indem z.B. pro Bewegungs-Richtung zwei Federn seriell oder parallel angeordnet sind. Hierdurch kann eine flachere Kennlinie bei serieller Anordnung oder eine flachere Kennlinie und höhere Endkraft bei paralleler Anordnung realisiert werden.

Figur 8 zeigt eine erste Ausführungsform eines automatischen Injektionsmechanismus, welche prinzipiell wie in den vorangehenden Figuren 1 bis 7 beschrieben funktional aufgebaut ist.

In der Perspektivansicht gemäß Figur 8A ist der automatische Insertionsmechanismus auf einer Infusionspumpe gezeigt, wobei der Insertionsmechanismus neben einem Reservoir 11 und einer Pumpe angeordnet ist, welche eine abzugebende Substanz in die oben beschriebene Hartkanüle 8 dosiert abgeben kann. In der gezeigten Ausführungsform sind sowohl Insertionsmechanismus, als auch der nachfolgend beschriebene automatisch lösbare Sicherungsmechanismus sowie die Pumpe zusammen mit dem Reservoir 11 auf einem gemeinsamen Substrat oder einer gemeinsamen Haltefläche angeordnet, welche beispielsweise an der Unterseite eine Haft- oder Klebefläche 12 aufweisen kann, um als so genannte Patch-Pumpe auf einen Körper aufgeklebt zu werden. Die Insertion der Kanüle 7, 8 erfolgt wie beschrieben durch Entspannung der Insertionsfeder 4, welche die Kanülen 7, 8 in Insertionsrichtung drückt oder schießt und für einen Ausstoß der Kanüle 7, 8 aus der Unterseite der Infusionspumpe sorgt. Die Kanüle 7, 8 wird durch eine Führung 6.1 in die gewünschte Ausstoßrichtung umgelenkt, welche von der Bewegungsrichtung der anderen Insertionskomponenten verschieden ist. Da sowohl Softkanüle 7 als auch Hartkanüle 8 biegbar oder flexibel ausgebildet sind, stellt dies kein Problem dar. Um ein Ausknicken nach oben zu verhindern, kann eine Führung am "Pod-Dach" z.B. oberhalb des Bogens im Bereich der Führung 6.1 vorgesehen sein.

Wie aus Figur 8C ersichtlich, drückt die distale in Figur 8C links gezeigte Seite des Soft-Kanülenhalters 2 durch den Druck der Insertionsfeder 4 auf den abgeschrägten Bereich 1.1a des Auslöseelements 1.1 des Kanülengehäuses 1, wodurch dieses Auslöseelement 1.1, welches als federnder Arm in einer Seitenwand des Kanülengehäuses 1 ausgebildet ist, in Freigaberichtung, wie durch den Pfeil P in Figur 8C eingezeichnet, mit einer Kraft beaufschlagt wird. Das Auslöseelement 1.1 ist über ein Verbindungselement 9.1 mit einer automatisch lösbaren Sicherung verbunden und beispielsweise fest mit einer Halterung 9.2a verbunden, an deren dem Auslöseelement 1.1 gegenüberliegenden Ende ein Schmelzelement 9.3 verbunden ist, das wiederum an dessen gegenüberliegendem Ende mit einer weiteren Halterung 9.2b verbunden ist, welche zum Beispiel fest und unbeweglich relativ zum Kanülengehäuse 1 oder zum Insertionsmechanismus beispielsweise mit dem Substrat der Patch-Pumpe verbunden ist. Das Schmelzelement 9.3 liegt somit zwischen den beiden Halterungen 9.2a und 9.2b und wird durch das Auslöseelement 1.1 und somit durch die Insertionsfeder 4 auf Zug belastet. Wäre das Schmelzelement 9.3 nicht vorhanden, könnte das Sperr- oder Auslöseelement 1.1 die durch die Insertionsfeder 4 verursachte Druckkraft des Soft-Kanülenhalters 2 nicht zurückhalten und die Insertionsbewegung oder Vorschubbewegung des Soft-Kanülenhalters 2 würde beginnen.

Verbindungselement 9.1, Halterungen 9.2a und 9.2b sowie Schmelzelement 9.3 bilden eine Schmelzsicherung 9.

In der Nähe des Schmelzelementes 9.3 ist zwischen elektrischen Kontakten, welche z.B. im Bereich von Ausfräsungen (10a und 10b) auf der Grundplatte sind, ein Erwärmungs- oder Heizelement 10 vorgesehen, welches zum Beispiel auf einem Steg zwischen den Ausfräsungen vorgesehen ist und z.B. in Gestalt eines Heizdrahtes oder einer Heizfläche ausgebildet sein kann und bei elektrischem Stromdurchfluss Wärme erzeugen kann. Hierzu kann beispielsweise die von der gezeigten Batterie 13 zur Verfügung gestellte elektrische Energie verwendet werden.

Soll eine automatische Insertion ausgelöst werden, so wird zum Beispiel Strom durch das Heizelement 10 geleitet, welches sich hierdurch erwärmt und welches durch diese Erwärmung das in der Nähe oder an dem Heizelement 10 vorgesehene Schmelzelement 9.3 aufweicht oder optional durchschmilzt, sodass zum Beispiel im Falle des Aufweichens oder im Falle der Schwächung des Materials des Schmelzelementes 9.3 oder auch im Falle des Durchschmelzens, also beispielsweise des Herbeiführens einer Unterbrechung oder einer Risses im Schmelzelement 9.3, wie beispielhaft in den Figuren 8F bis 8H gezeigt, die mechanische Kopplung oder Halteverbindung zwischen den Halterungen 9.2a und 9.2b der Schmelzsicherung unterbrochen oder aufgehoben wird. Hierdurch wird die auf Zug belastete Halterung 9.2a nicht mehr von dem Schmelzelement 9.3 an der Halterung 9.2b gehalten und kann somit auch das mit dem Auslöseelement 1.1 verbundene Verbindungselement 9.1 nicht mehr halten, sodass das Auslöseelement 1.1 durch die Kraft der Insertionsfeder 4 aus der Sperrposition in Freigabeposition gedrückt wird, wie in den Figuren 8F bis 8J gezeigt. Die Insertionsfeder 4 kann sich somit entspannen und wie oben beschrieben den Insertionsvorgang auslösen.

Außer der Insertionsfeder 4 ist für die erfindungsgemäße Aufweich- oder Schmelzsicherung kein weiterer Kraftspeicher oder kein weiteres Federelement erforderlich, um eine elektrisch ansteuerbare automatische Insertionsauslösung zu realisieren. Auf weitere Federelemente kann somit erfindungsgemäß verzichtet werden, was einen einfachen und störungsunanfälligen Aufbau eines automatischen Insertionsmechanismus ermöglicht.

Nach erfolgter Insertion der Softkanüle 7 kann, wie oben beschrieben, die Hartkanüle 8 innerhalb der Softkanüle 7 wieder zurückgezogen werden, sodass die ausgeschobene Softkanüle 7 im ausgeschobenen Zustand verbleibt, wie in den Figuren 8K bis 8O gezeigt.

Anschließend kann der Abgabe einer Substanz aus dem Reservoir oder der Pumpe 11 begonnen werden, welche die Substanz dosiert an die Hartkanüle 8 abgibt, von welcher diese an die Softkanüle 7 abgegeben und an dem distalen Ende der Softkanüle 7 abgegeben und zum Beispiel injiziert wird.

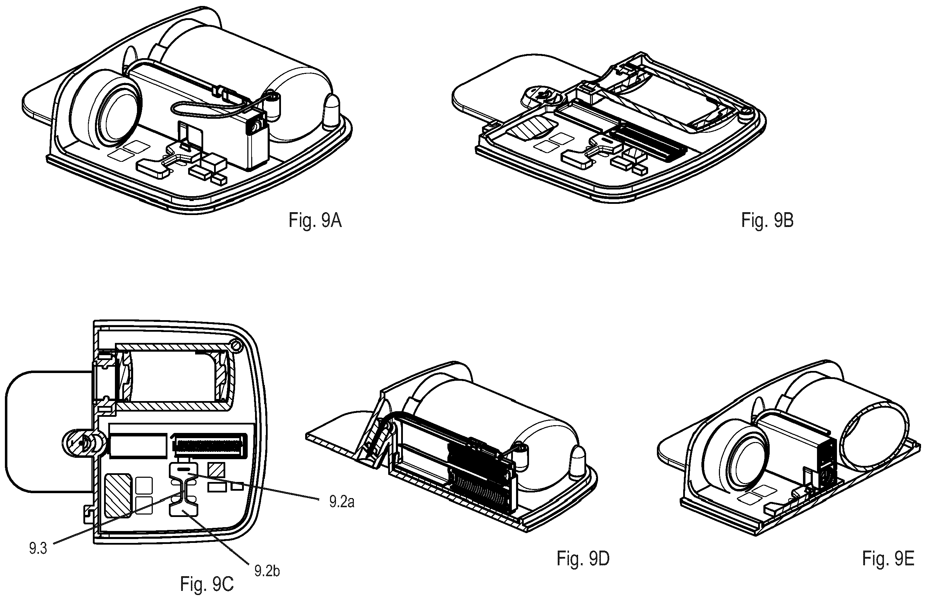

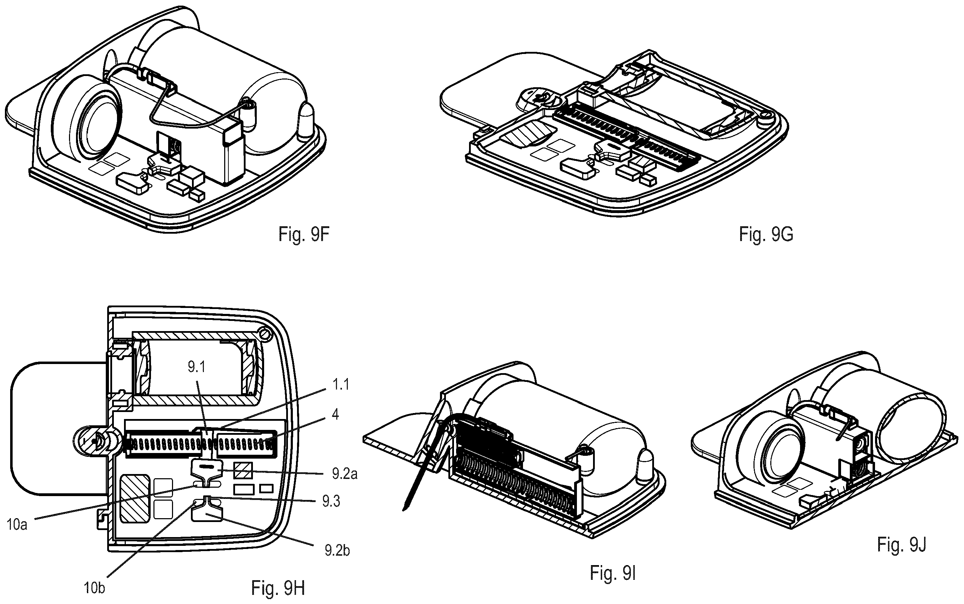

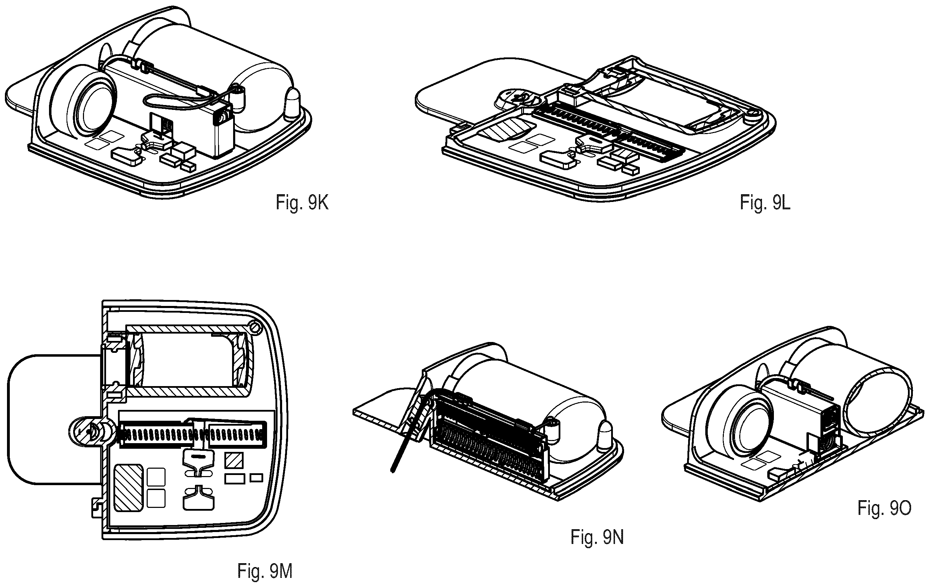

Figur 9 zeigt ein zweites Ausführungsbeispiel einer Aufweichsicherung oder Schmelzsicherung, welche prinzipiell gleich zur Ausführungsform gemäß Figur 8 aufgebaut ist, sofern nachfolgend nichts anderes beschrieben ist. Auf die Beschreibung zur Figur 8 wird somit vorwiesen, welche zur Vereinfachung hier nicht nochmals wiederholt wird.

Das Schmelzelement 9.3 ist wiederum zwischen Halterungen 9.2a und 9.2b angeordnet, abweichend vom Ausführungsbeispiel der Figur 8 hier jedoch als leitender Kunststoff ausgebildet.

Zur Auslösung oder Freigabe des Insertionsmechanismus wird ein Stromfluss durch den leitenden Kunststoff 9.3 verursacht, wodurch dieser erwärmt wird und entweder soweit geschwächt wird, dass die von der Insertionsfeder 4 herrührende Zugkraft das Schmelzelement 9.3 durchtrennt, wie in den Figuren 9F bis 9J gezeigt. Alternativ oder ergänzend kann das Durchtrennen des Schmelzkörpers 9.3 auch vollständig durch die Erhitzung durch den erwähnten Stromfluss erfolgen.

Der leitende Kunststoff kann von elektrischen Kontakten mit einer Spannungsdifferenz beaufschlagt werden, sodass hierdurch gemäß Ohm'schem Gesetz ein Stromfluss durch das Schmelzelement 9.3 verursacht wird.

Alternativ kann das Schmelzelement 9.3 auch mit zwei seitlichen Elektroden versehen sein, welche jeweils entlang der Längsrichtung des Schmelzelementes 9.3 verlaufen, sodass bei Anlegen einer Potentialdifferenz zwischen diesen Elektroden die durch das Schmelzelement 9.3 beabstandeten Elektroden einen Strom und somit eine Erwärmung des Schmelzelementes 9.3 in Form eines leitenden Kunststoffes verursachen, wodurch dieses geschwächt oder durchtrennt wird, um die Sicherung freizugeben.

In den in den Figuren 8 und 9 gezeigten Ausführungen ist es vorteilhaft, wenn das Schmelzelement 9.3 zum Beispiel verjüngt ist oder einen relativ kleinen Querschnitt aufweist, welcher ausreichend breit oder stark sein sollte, um die Haltekraft für das Auslöseelement 1.1 zum Halten der Sicherung zur Verfügung zu stellen. Eine Verjüngung oder Reduzierung des Querschnitts des Schmelzelementes 9.3 ermöglicht ein einfaches Schmelzen oder Öffnen der Sicherung.

Figur 10 zeigt eine dritte Ausführungsform, welche prinzipiell identisch wie die zuvor beschriebenen Ausführungsformen aufgebaut ist, sofern nicht anders beschrieben.

Das Auslöseelement 1.1 des Kanülengehäuses 1 wird durch die Insertionsfeder 4 aufgrund der Abschrägung 1.1a in seitliche Richtung gedrückt, also zum Beispiel orthogonal zur Längsrichtung der Insertionsfeder 4. Ein Aufweichelement oder Schmelzelement 9.4 verhindert, dass das Auslöseelement 1.1 aus der Sperrstellung in die Freigabestellung gelangen kann. Das Schmelzelement 9.4 kann zum Beispiel stabförmig oder als länglicher Körper ausgebildet sein. Beispielsweise kann das Schmelzelement 9.4 aus einem aufweichbaren oder schmelzbaren Material bestehen.

In der Nähe des Schmelzelementes 9.4 ist ein Erwärmungselement oder Heizelement 10 vorgesehen, welches das Schmelzelement 9.4 zumindest zum Teil erwärmen oder schmelzen kann, um dessen Haltekraft gegen die Kraft des in Freigabestellung drückenden Auslöseelementes 1.1 zu schwächen oder aufzuheben. Im in Figur 10C gezeigten Ausführungsbeispiel kann das Heizelement 10 im Bereich des Schmelzelementes 9.4 vorgesehen sein, welches dem Bereich gegenüberliegt, der mit dem Auslöseelement 1.1 in Kontakt steht. Es ist auch möglich das Heizelement 10 an einer anderen Stelle vorzusehen, beispielsweise im Bereich des Kontaktes zu dem Auslöseelement 1.1 oder im Bereich der Führungselemente 14, welche ein Verschieben des Schmelzelementes 9.4 in axialer Richtung ermöglichen.

Wird das Heizelement 10 erwärmt, zum Beispiel indem es als elektrisches Heizelement ausgebildet ist und mit Strom durchflossen wird, so weicht das Schmelzelement 9.4 auf oder schmilzt und kann dem von dem Auslöseelement 1.1 ausgeübten Druck nicht länger standhalten, sodass zum Beispiel das Schmelzelement 9.4 aufgrund eines teilweisen Schmelzvorganges verkürzt wird, wodurch das Halteelement 1.1 aus der Sperrstellung in eine Freigabestellung gedrückt wird, beispielsweise in Figur 10H gezeigt. Hierdurch kann die Insertion ausgelöst werden.

Ein Schmelzelement kann in allen Ausführungsformen zum Beispiel aus einem thermoplastischen Material, wie zum Beispiel einem Thermopolymer bestehen und bei Erwärmung teilweise oder vollständig geschmolzen werden, wobei die flüssige Phase des Materials als auch Gleit- oder Schmiermittel dienen kann, sodass sich ein optional verbleibender fester Teil des Schmelzelementes beispielsweise leichter innerhalb einer optional vorgesehenen Führung bewegen kann.

Figur 11 zeigt ein viertes Ausführungsbeispiel, welches ähnlich wie das dritte Ausführungsbeispiel aufgebaut ist.

Anstelle des Schmelzelementes 9.4 des dritten Ausführungsbeispiels ist ein Stößel 15 vorgesehen, welcher aufweichbar oder schmelzbar ausgestaltet sein kann oder alternativ als nicht schmelzbares oder nicht aufweichbares Element ausgestaltet ist. Der Stößel 15 ist wiederum in Führungselementen 14 axial verschiebbar geführt und hält das auf den Stößel 15 drückende Sperrelement 1.1 in Sperrstellung. An der dem Sperr- oder Auslöseelement 1.1 gegenüberliegenden Stelle des Stößels 15 stützt sich der Stößel 15 an einem aufweichbaren oder schmelzbaren Halteelement oder Plättchen 9.5 ab, welches beispielsweise aus einem schmelzbaren oder aufweichbaren Kunststoff oder einem leitfähigen Kunststoff oder einem Metall gebildet wird. Wird das Halteelement 9.5 z.B. von einem Heizelement erwärmt oder beispielsweise von einem Strom durchflossen, sofern es leitfähig ausgebildet ist, erwärmt sich das Halteelement 9.5, welches in Ausgangsstellung zum Beispiel fest und unbeweglich relativ zum Kanülengehäuse 1 vorgesehen ist und verliert so die Fähigkeit den Stößel 15 gegen die von dem Auslöseelement 1.1 wirkende Druckkraft abzustützen. Figur 11H zeigt den Zustand nach dem Auslösen, also beispielsweise partiellen Wegschmelzen oder Aufweichen des Halteelementes 9.5, wodurch das Auslöseelement 1.1 aus der Sperrstellung in die Freigabestellung gelangen kann und eine nachfolgende Insertion erfolgen kann.

Gemäß einer Ausführungsform kann das Halteelement 9.5 ein festes Element sein, welches durch eine Löt- oder Klebeverbindung an einem Gehäuse, z.B. des Infusionsgerätes, befestigt ist. Diese Befestigung kann durch Erwärmung gelöst werden.

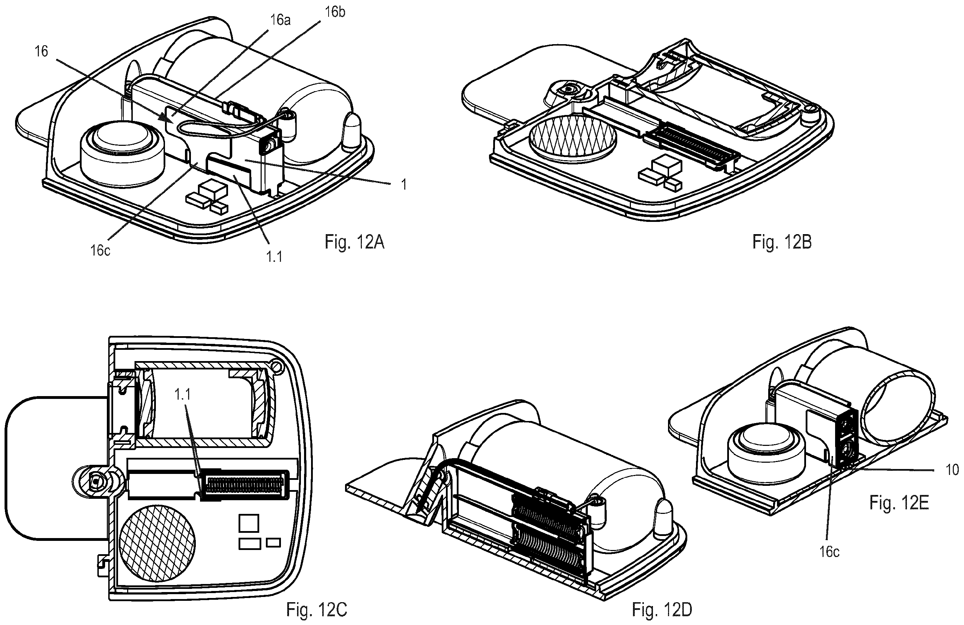

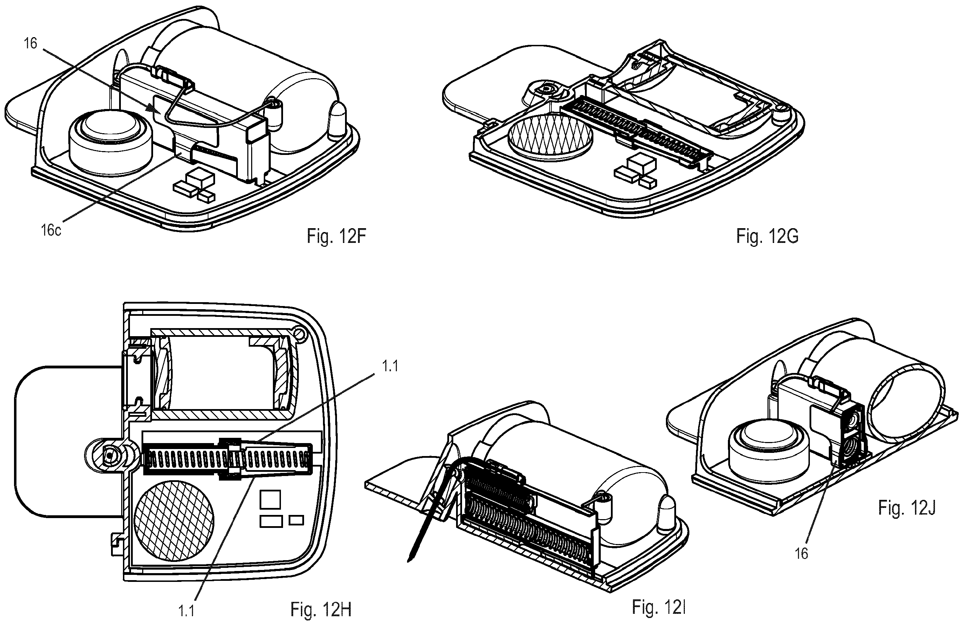

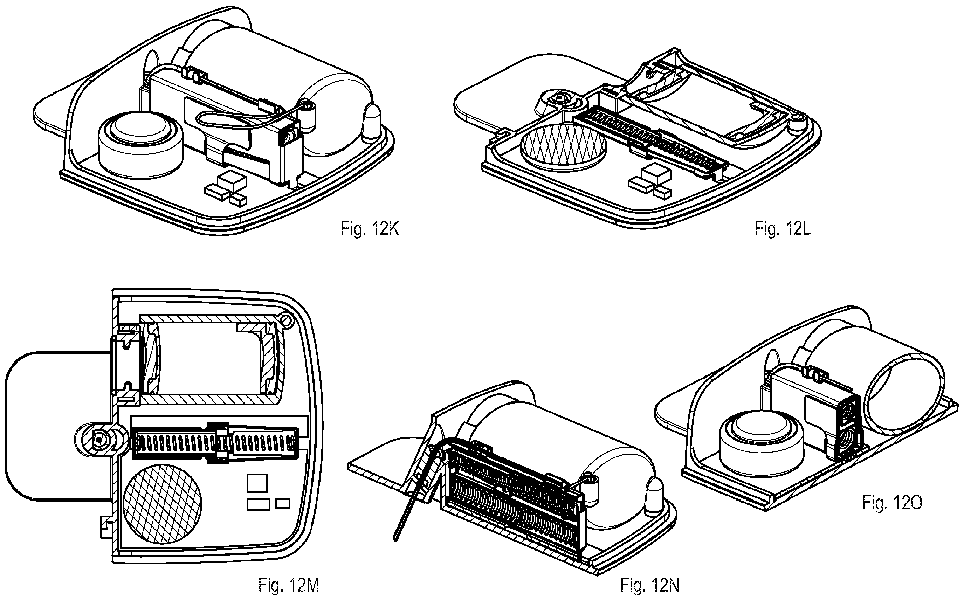

Figur 12 zeigt ein fünftes Ausführungsbeispiel, wobei eine Folie 16 oder ein flächiges Element auf dem oder den Auslöseelementen 1.1 vorgesehen ist, um das oder die Auslöseelemente 1.1 im Ausgangszustand in einer Sperrposition zu halten. Die Folie 16 kann beispielsweise über das oder die Auslöseelemente 1.1 geklebt oder gespannt werden. Beispielsweise kann die Folie 16 mit einer Gehäuseseite des Kanülengehäuses 1 verklebt oder anderweitig daran befestigt, zum Beispiel verschweißt oder eingespannt, sein und durch das vorzugsweise an mehreren Stellen der Folie 16 realisierte Aufbringen oder Befestigen der Folie 16 an dem Kanülengehäuse 1 kann die Folie 16 das oder die Sperrelemente 1.1 in Sperrstellung halten.

Im in Figur 12 gezeigten Ausführungsbeispiel ist die Folie beispielsweise als Doppel-T-Struktur ausgebildet, wobei zwei verbreiterte Aufbringbereiche oder Klebebereiche 16a und 16b an gegenüberliegenden Enden der Folie 16 vorgesehen sind, welche durch einen schmäleren oder verjüngten Folienmittelbereich 16c verbunden sind. Die Folie 16 kann mit den breiten Anbringbereichen 16a und 16b beispielsweise an gegenüberliegenden Seitenflächen des Kanülengehäuses 1 angebracht oder befestigt oder aufgeklebt werden, sodass sich der schmälere Verbindungsbereich 16c über das oder die Auslöseelemente 1.1 erstreckt und diese in Sperrposition hält, wie in Figur 12A gezeigt.

Ein Heizelement 10 kann an oder in der Nähe der Folie 16 vorgesehen sein, beispielweise am oder im Bereich des schmäleren Folienbereiches 16c und zur Erwärmung der Folie 16 dienen. Die Folie 16 kann beispielsweise aus einem Kunststoff oder einem anderen Material bestehen, welcher durch Erwärmung in seiner mechanischen Halte- oder Tragfähigkeit geschwächt werden kann, sodass nach der Erwärmung der Folie 16 durch das Heizelement 10 die Folie 16 entweder aufweicht oder aufweitet oder optional auch reißt, wodurch die Haltekraft der Folie 16 zum Halten der aus der Sperrstellung in Freigabestellung drückenden Auslöseelemente 1.1 geschwächt wird, sodass, wie in Figuren 12F, 12H und 12J gezeigt, die Folie 16 die Halteelemente 1.1 nicht mehr in Sperrstellung halten kann, sodass diese in Freigabestellung gelangen und die Insertion ausgelöst wird.

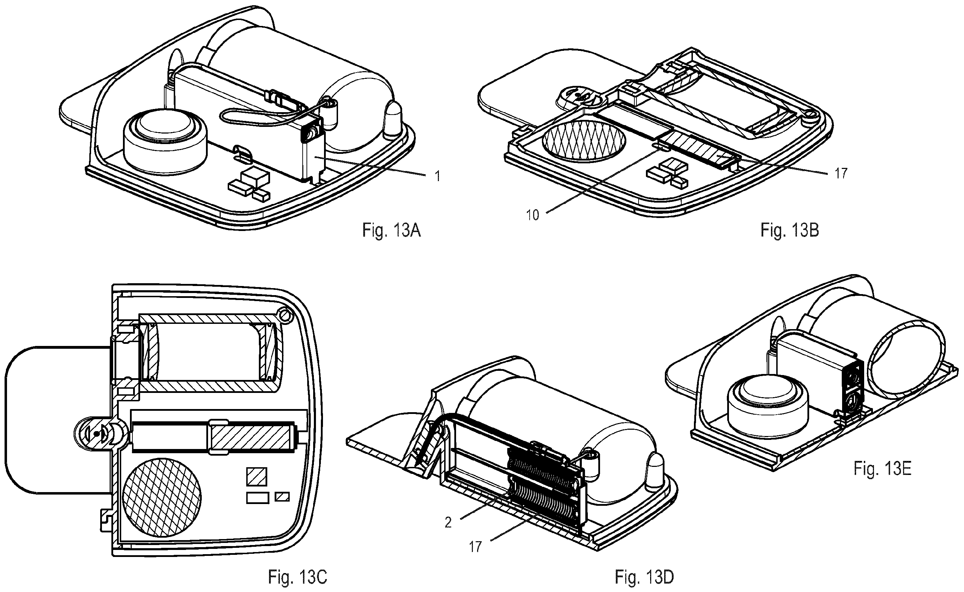

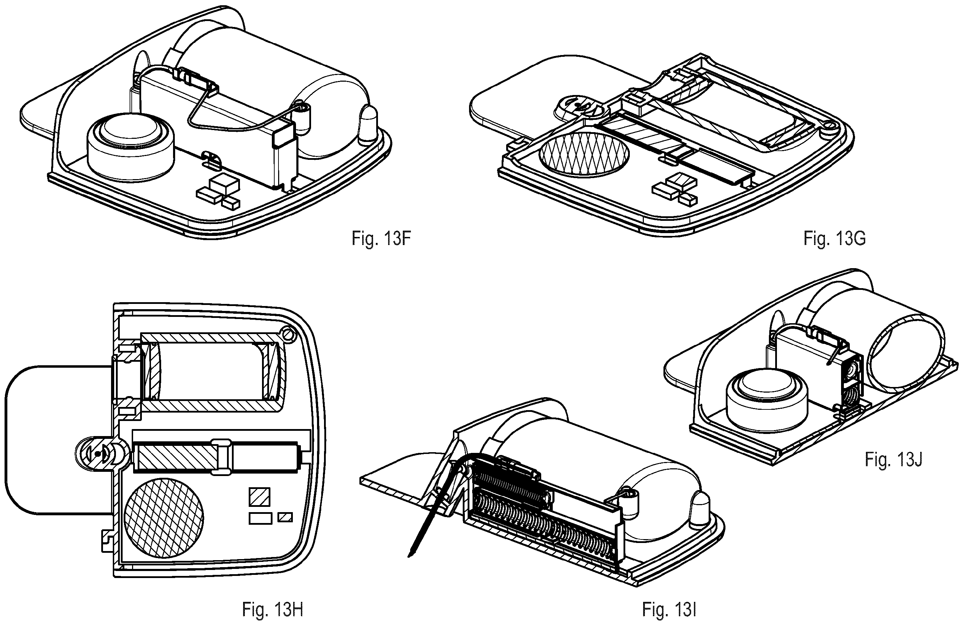

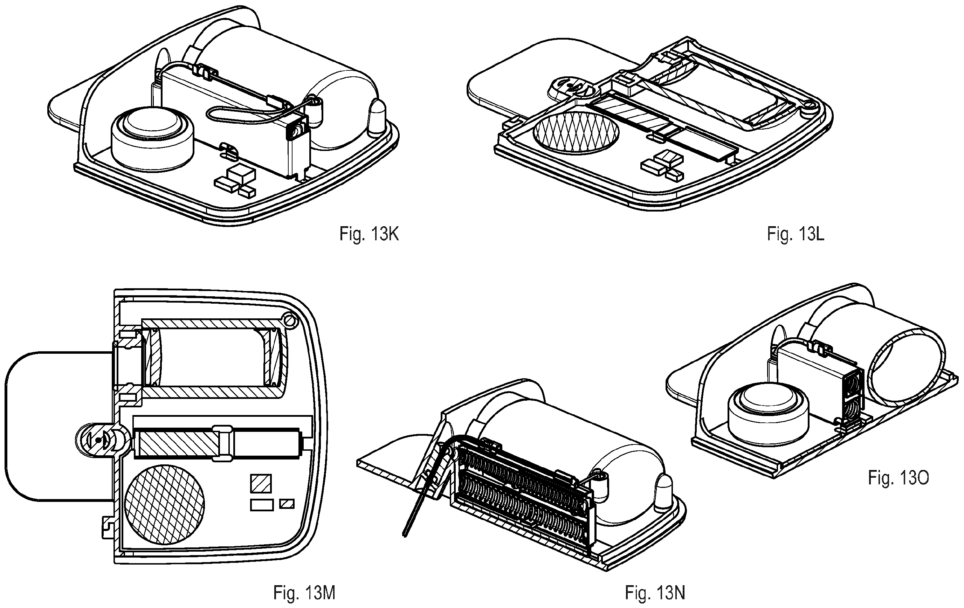

Figur 13 zeigt ein sechstes Ausführungsbeispiel einer automatisch lösbaren Sicherung, wobei anders als in den vorangehenden Ausführungsbeispielen keine Auslöseelemente 1.1 vorgesehen sind. Stattdessen wird der Soft-Kanülenhalter 2 relativ zum Nadelgehäuse 1 gegen die Kraft der Insertionsfeder 4 dadurch in Sperrposition gehalten, dass der Soft-Kanülenhalter 2 relativ unverschiebbar zum Kanülengehäuse 1 angeklebt ist. Die Haft- oder Klebefläche 17 zum Halten des Soft-Kanülenhalters 3 kann zum Beispiel am Kanülengehäuse 1 oder alternativ oder zusätzlich auf dem Substrat der Patch-Pumpe vorgesehen sein.

Die Klebefläche 17 wird mittels eines Klebematerials realisiert, welches seine Klebekraft oder Haltekraft bei der Erwärmung verringert, sodass zur Auslösung der Insertion das Klebematerial mittels eines Erwärmungs- oder Heizelementes 10, beispielsweise eines elektrischen Heizelementes 10, welches zum Beispiel am oder in der Nähe der Haft- oder Klebefläche 17 vorgesehen ist, aufgeweicht werden kann. Hierdurch kann das ortsfeste Halten des Soft-Kanülenhalters 2 geschwächt oder aufgelöst werden, bis die durch die Insertionsfeder 4 wirkende Druckkraft auf den Soft-Kanülenhalter 2 größer ist als die Haltekraft der Haft- oder Klebefläche 17, sodass sich der Soft-Kanülenhalter 2 relativ zum Gehäuse angetrieben durch die Insertionsfeder 4 bewegen kann, wodurch eine Insertion ausgelöst wird, wie in Figuren 13F bis 13J gezeigt.

Nachfolgend werden Sicherungs- und Auslösemechanismen beschrieben, welche für einen Insertionsmechanismus verwendet werden können, wie beispielsweise in der Ausführungsform gemäß den Figuren 1 bis 7 beschrieben.

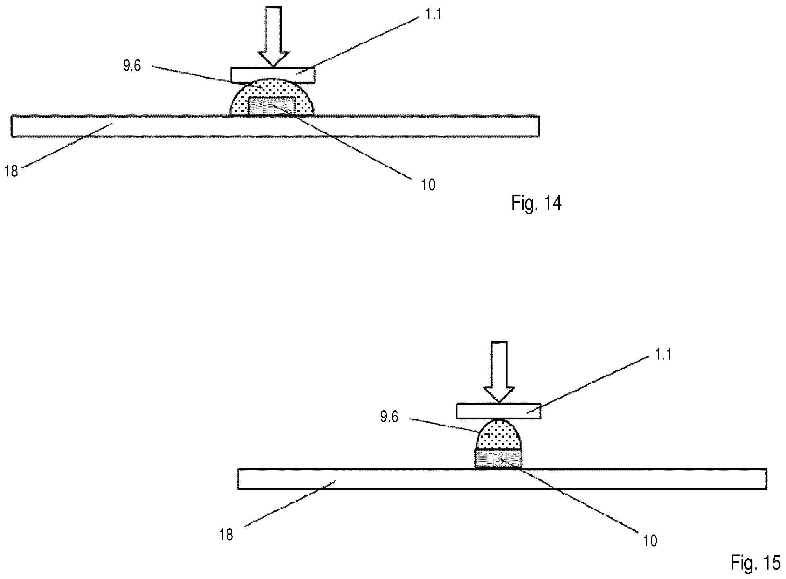

Figur 14 zeigt eine Ausführungsform mit einem auf Druck belasteten schmelzbaren Medium 9.6, welches zwischen einem Heizelement 10 und einem Auslöseelement 1.1, z.B. wie vorhergehend beschrieben, angeordnet sein kann. Das Auslöseelement 1.1 kann mit einer Dauerbelastung beaufschlagt sein, welche z.B. durch die Insertionsfeder 4 verursacht wird, wie symbolisch durch den Pfeil dargestellt. In der gezeigten Ausführungsform umgibt das schmelzbare Medium 9.6, welches in einem festen Zustand vorliegt, das Heizelement 10 zumindest an der Oberseite und in der gezeigten Ausführungsform auch an den seitlichen Flächen, wobei das Heizelement 10 auf einem Basiselement oder Substrat 18 angeordnet ist.

In dieser Ausführungsform könnte das schmelzbare Material oder Medium 9.6 beispielsweise ein Heissleim sein. Ein Toleranzausgleich findet automatisch statt.

Wird das Heizelement 10 erwärmt, so wird das schmelzbare Medium 9.6, wie beispielsweise ein Heissleim, erwärmt oder aufgeweicht, wodurch das durch die Dauerbelastung (angedeutet durch den Pfeil in Figur 14) belastete Auslöseelement 1.1 das schmelzbare oder aufweichbare Medium 9.6 in Richtung auf das Heizelement 10 drücken kann und somit beispielsweise einen Sperrzustand aufheben kann, sodass beispielsweise eine wie oben beschriebene Insertion freigegeben oder ausgelöst werden kann.

Figur 15 zeigt eine weitere Ausführungsform ähnlich zur in Figur 14 beschriebenen Ausführungsform, wobei das schmelzbare Medium 9.6 auf dem Heizelement 10 vorgesehen ist, dieses jedoch nicht an den Seitenflächen umgibt oder abdeckt. Beispielsweise könnte das schmelzbare Medium 9.6 ein Lötzinn oder wie oben erwähnt ein Heissleim sein und beispielsweise auch eine Klebeeigenschaft aufweisen, um beispielsweise das Auslöseelement 1.1 im ausgelösten Zustand zu halten.

Die Auslösung erfolgt wie oben beschrieben durch Erwärmung des Heizelements 10 und Aufweichen oder Schmelzen des schmelzbaren oder aufweichbaren Mediums 9.6, wodurch das Auslöseelement 1.1 durch die z.B. von der Insertionsfeder 4 oder einem anderen Mechanismus herrührende Dauerbelastung aus der in Figur 15 gezeigten Sicherungsposition in Richtung auf das Heizelement 10 gedrückt oder verschoben werden kann, um eine Sperrstellung aufzulösen.

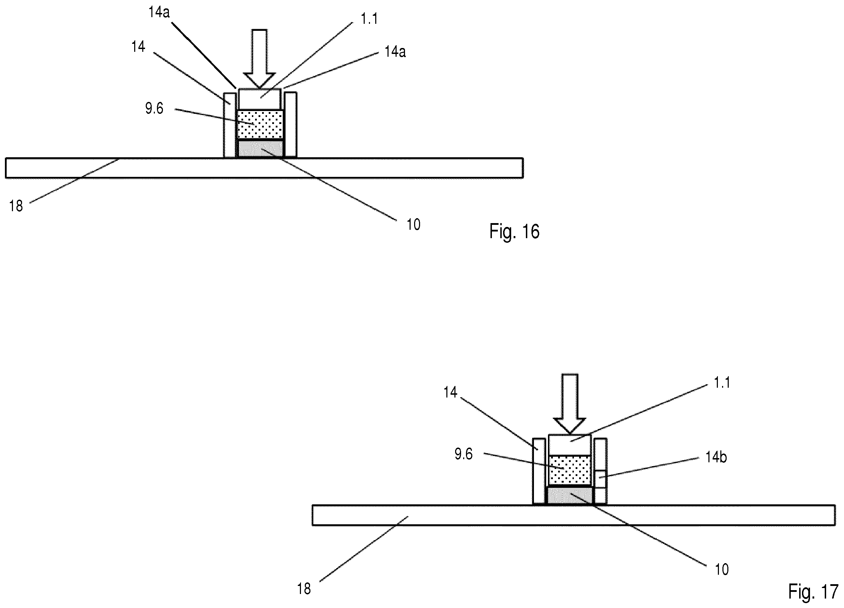

Figur 16 zeigt eine Ausführungsform, bei welcher eine Kraft oder Dauerbelastung (angedeutet durch den Pfeil) auf ein Auslöseelement 1.1 oder einen Stößel wirkt, welcher zumindest partiell oder vollständig in einer Führung oder einem Hohlzylinder 14 auf einer Seite eines aufweichbaren oder schmelzbaren Mediums 9.6 angeordnet ist, das von der Führung oder dem Hohlzylinder 14 umgeben wird und welches an der dem Auslöseelement 1.1 gegenüberliegenden Seite an einem Heizelement 10 anliegt. Am seitlichen Rand des Auslöseelements 1.1 ist eine Abflussöffnung 14a vorgesehen, welche zwischen dem Auslöseelement 1.1 und der Führung oder dem Hohlzylinder 14 gebildet wird.

Wird das Heizelement 10 erwärmt, so kann das schmelzbare Medium 9.6 schmelzen und durch die Abflussöffnung 14a austreten, wenn es bedingt durch die auf das Auslöseelement 1.1 wirkende Dauerbelastung mit Druck beaufschlagt wird, wodurch das Auslöseelement 1.1 in Richtung auf das Heizelement 10 von einer Sperrstellung in eine Freigabestellung bewegt wird.

Die bei dieser Ausführungsform benötigte Heizenergie ist im Vergleich zu den vorangehend beschriebenen beiden Varianten höher, da mehr Material erwärmt werden muss. Für das schmelzbare Medium 9.6 können weichere Materialien verwendet werden, da dieses durch die Führung oder den Hohlzylinder 14 eingeschlossen ist. Das schmelzbare Material 9.6 ist z.B. ein nicht klebendes Material und ist beispielsweise ein langsam erkaltendes Material, damit dieses Material nicht erstarrt, bevor es durch die Abflussöffnung 14a abfließen kann.

Figur 17 zeigt eine weitere Ausführungsform, welche ähnlich wie in Figur 16 aufgebaut ist, wobei die Abflussöffnung 14b nicht durch einen Spalt zwischen Stößel oder Auslöseelement 1.1 und Führung 14 gebildet wird, sondern stattdessen direkt in der Führung 14 vorgesehen ist, z.B. auf einer Höhe der Führung 14, bei welcher das Heizelement 10 an das schmelzbare Medium 9.6 angrenzt.

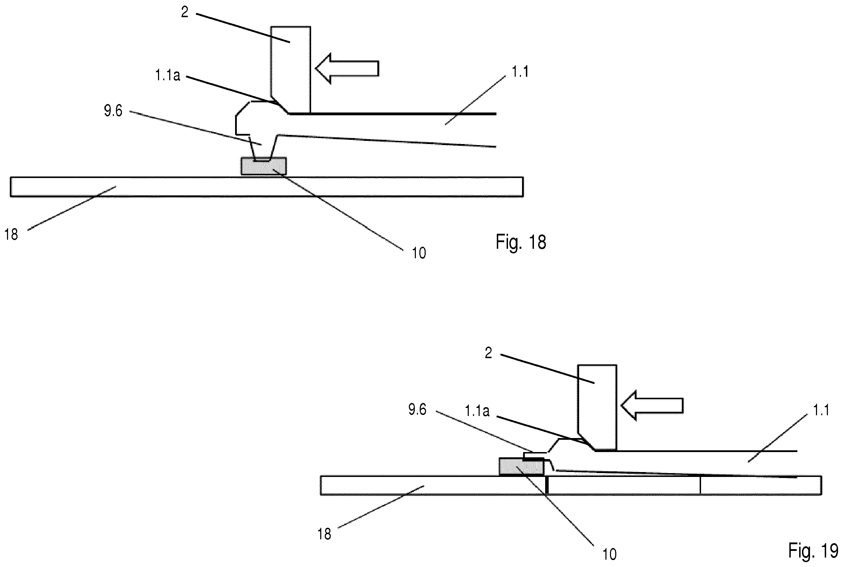

Figur 18 zeigt eine weitere Ausführungsform, bei welcher die Kraft auf das Sperrelement durch Umlenkung reduziert oder untersetzt werden kann. Dabei kann ein mit 2 bezeichnetes Element, wie beispielsweise ein Soft-Kanülen-Halter, aus der durch den Pfeil angezeichneten Richtung mit einer Dauerbelastung oder Kraft beaufschlagt werden, welche an einer Antriebsfläche oder Schräge 1.1a eines Auslöseelements 1.1 anliegt und dieses quer oder senkrecht zur als Dauerbelastung wirkenden Kraft mit Druck beaufschlagt. Das Auslöseelement 1.1 enthält eine zu schmelzende Struktur oder ein schmelzbares Medium 9.6, welches an einem Heizelement 10 anliegt. Wird das Heizelement 10 erwärmt, so wird das schmelzbare oder aufweichbare Medium 9.6 aufgeweicht oder geschmolzen, wodurch das Auslöseelement 1.1 bedingt durch die auf das Element 2 wirkende Kraft von einer Sperrstellung in eine Freigabestellung bewegt wird und einen Insertionsvorgang freigeben oder auslösen kann.

Figur 19 zeigt eine Ausführungsform, bei welcher ein mit 2 bezeichnetes Element, wie beispielsweise ein Soft-Kanülen-Halter, z.B. von einer Insertionsfeder mit einer Kraft beaufschlagt wird und durch die eingezeichnete Antriebsfläche oder Schräge 1.1a auf ein Auslöseelement 1.1 wirkt und dieses in seitlicher Richtung oder Freigaberichtung mit einer Kraft beaufschlagt wird. Das Auslöseelement 1.1 weist ein schmelzbares Medium 9.6 auf, welches in der gezeigten Ausführungsform sowohl mit einer Druckkraft als auch mit einer Scherkraft beaufschlagt wird. Wird das schmelzbare Medium 9.6 aufgeweicht oder geschmolzen, z.B. durch Erwärmung des Heizelements 10, kann das Auslöseelement 1.1 aus der in Figur 19 gezeichneten Sperrstellung in eine Freigabestellung quer oder senkrecht zu der als Dauerbelastung wirkenden Kraft gedrückt werden und so eine Insertion freigeben oder auslösen.

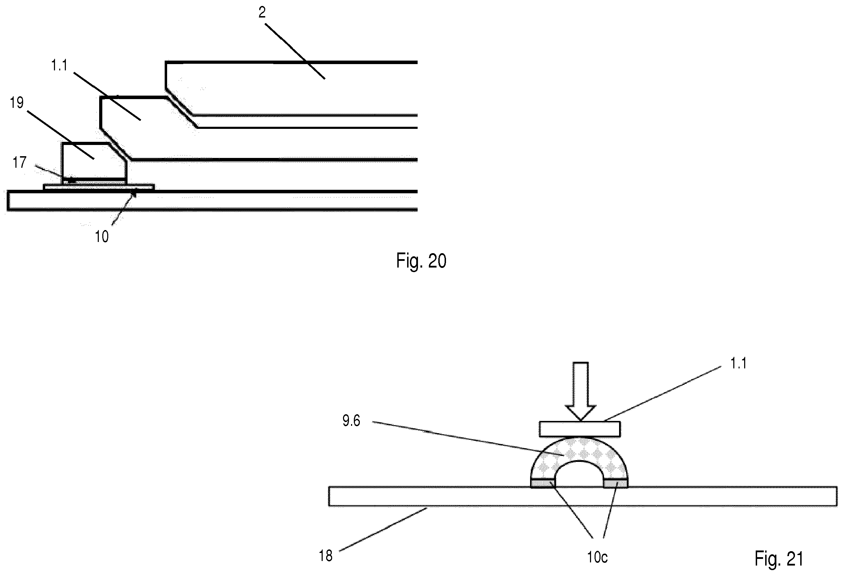

Figur 20 zeigt eine Ausführungsform ähnlich zu der in Figur 13 gezeigten Ausführungsform, bei welcher eine Haftfläche oder Klebefläche 17 auf einem Heizelement 10 vorgesehen ist und anders als in der Ausführungsform gemäß Figur 13 gezeigt, nicht direkt an einem zu haltenden Element, wie z.B. dem Soft-Kanülen-Halter 2 anliegt, sondern z.B. direkt oder mittels einer darauf aufgeklebten Struktur 19 ein Sperrelement in einer Sperrstellung hält. Das Sperrelement wird durch das zu haltende Element oder den Soft-Kanülen-Halter 2 mit einer Kraft beaufschlagt und drückt seinerseits auf die von der Haftfläche oder Klebefläche 17 gehaltene Struktur. Wird die Haftfläche oder Klebefläche 17 durch das Heizelement 10 geschwächt oder geschmolzen, so kann die damit verbundene Struktur von dem Sperrelement weggedrückt werden, wobei auf der Klebestelle eine Schubbelastung sowie eine Druckbelastung wirkt. Das Sperrelement kann somit von dem zu haltenden Element 2 in Freigabestellung gedrückt werden, wodurch eine Insertion ausgelöst werden kann.

Figur 21 zeigt eine Ausführungsform, bei welcher Sperr- und Heizelemente kombiniert sind. Hierdurch kann ermöglicht werden, dass eine Erwärmung oder Hitze direkt wirkt, was die Effizienz erhöhen kann.

Ein Auslöseelement 1.1, wie z.B. das zuvor beschriebene Sperr- oder Rückhalteelement oder Auslöseelement-Inserter, wird dauerbelastet wie durch den Pfeil angedeutet und drückt seinerseits auf ein schmelzbares oder erweichbares elektrisch leitendes Medium 9.6, welches zwischen zwei elektrischen Kontakten 10c vorgesehen ist. Wird zwischen den elektrischen Kontakten 10c eine Spannung angelegt, so fließ durch das schmelzbare und elektrisch leitende Medium 9.6 ein Strom, welcher zur Erwärmung des Mediums 9.6 führt und eine Erweichung oder ein Schmelzen oder eine Schwächung des Mediums 9.6 herbeiführt, sodass das Auslöseelement 1.1 in Richtung des Mediums 9.6 oder in Richtung auf den elektrischen Kontakt 10c von einer Sperrstellung in eine Freigabestellung gedrückt werden kann.

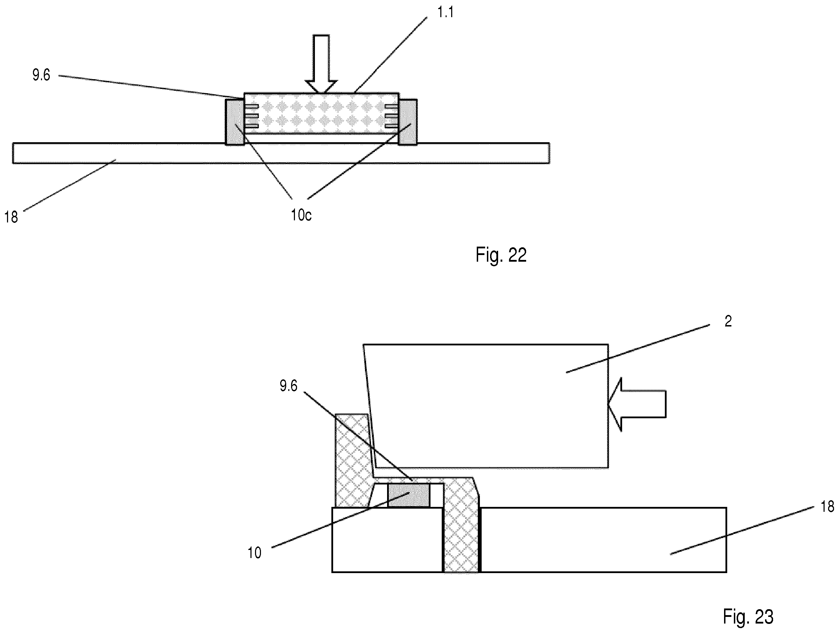

Figur 22 zeigt eine Ausführungsform, bei welcher ein Auslöseelement 1.1 auf ein schmelzbares elektrisch leitendes Medium 9.6 drückt, welches zwischen elektrischen Kontakten 10c vorgesehen ist. Das Medium 9.6 kann z.B.

ein leitender Kunststoff beispielsweise PreElec- PP1380sein und wird zur Freigabe des Auslöseelements 1.1 mit einer zwischen den elektrischen Kontakten 10c anlegenden elektrischen Spannung beaufschlagt, wodurch in dem Medium 9.6 ein Strom fließt und dieses aufweicht.

Figur 23 zeigt eine Ausführungsform, bei welcher ein Element 2, wie z.B. ein Soft-Kanülen-Halter, mit Druck beaufschlagt wird und durch ein schmelzbares Medium 9.6 gehalten wird, welches durch ein Heizelement 10 geschwächt oder aufgeweicht oder geschmolzen werden kann. Dabei wird das schmelzbare Medium 9.6 im Erwärmungsbereich oder in der Nähe des Heizelements 10 auf Zug beansprucht, sodass relativ wenig Material oder Volumen des schmelzbaren Mediums 9.6 erhitzt werden muss, da die Belastung im Medium oder Material 9.6 immer größer wird, je kleiner der Querschnitt wird.

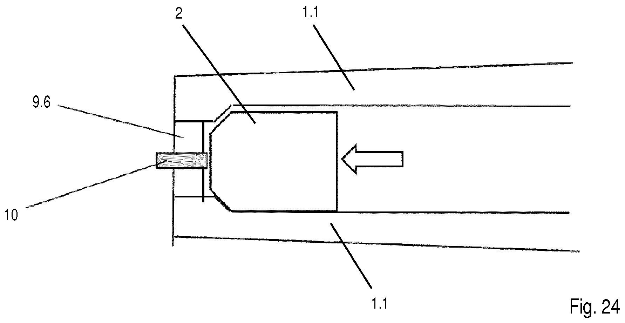

Figur 24 zeigt eine Ausführungsform bei welcher ein schmelzbares oder aufweichbares oder schwächbares Medium 9.6 ähnlich einer Sollbruchstelle an einem oder zwei Auslöseelementen 1.1 vorgesehen ist und dieses oder diese in einer Sperrstellung hält. In der gezeigten Ausführungsform ist ein Heizelement 10 innerhalb des schmelzbaren Mediums 9.6 vorgesehen. Auf jedes Auslöseelement 1.1 wirkt eine Dauerbelastung durch das Element 2, wie z.B. einen Soft-Kanülen-Halter, welcher auf das oder die Auslöseelemente 1.1 drückt, welche von dem schmelzbaren Medium 9.6 in Sperrstellung gehalten werden. Wird das Heizelement 10 erwärmt, so wird das schmelzbare Medium 9.6 aufgeweicht, geschwächt oder geschmolzen, sodass die Auslöseelemente 1.1 das Element 2 nicht mehr halten können, wodurch eine Insertion freigegeben oder ausgelöst werden kann.

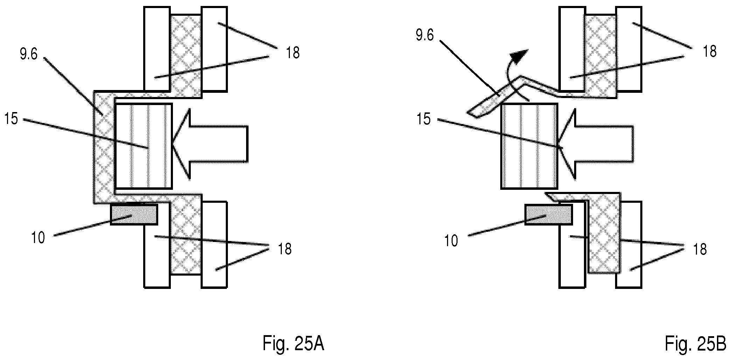

Figur 25A zeigt eine Ausführungsform ähnlich zu der in Figur 11 beschriebenen Ausführungsform. Dabei wird ein schmelzbares oder aufweichbares oder schwächbares Element 9.6 durch einen Stößel 15, welcher wie z.B. in Figur 11C gezeigt, einen Soft-Kanülen-Halter 2 in Sperrstellung hält, mit einer Kraft beaufschlagt und drückt auf das schmelzbare Element 9.6, wie in Figur 25A gezeigt.

Wird das schmelzbare Element 9.6 durch Erwärmung des Heizelements 10 geschwächt oder aufgeweicht oder geschmolzen, so kann dieses der Belastung durch den Stößel 15 nicht mehr standhalten, wodurch der Stößel 15 verschoben wird und eine Insertion freigeben kann, wie in Figur 25B gezeigt. Die Haltekraft wird beim Element 9.6 auf zwei Stege verteilt. Das Erweichen findet nur auf einem dieser beiden Stege statt, was den Erwärmungsbereich reduzieren kann und damit die Effizienz steigern kann.

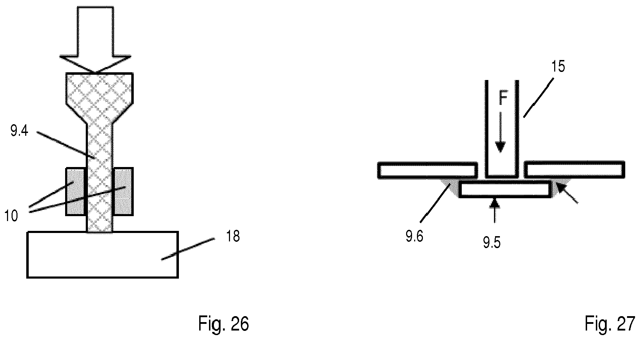

Figur 26 zeigt eine Ausführungsform ähnlich zu der in Figur 10 gezeigten Ausführungsform, bei welcher ein schmelzbares Element 9.4 auf Druck belastet ist und - anders als in der Ausführungsform gemäß Figur 10 gezeigt - von seitlichen Heizelementen 10 erwärmt wird, welche z.B. im Bereich der im Ausführungsbeispiel gemäß Figur 10 gezeigten Führung 14 vorgesehen sein können. Wird das schmelzbare Element 9.4 durch das oder die Heizelemente 10 erwärmt, so kann das erweichte oder geschmolzene Material seitlich zu dem oder den Heizelementen 10 entweichen, z.B. im Zwischenraum zwischen Heizelement 10 und Basiselement 18. Bei der gezeigten Ausführungsform wird ein Heizelement 10 mechanisch nur relativ wenig belastet.

Figur 27 zeigt eine Ausführungsform des Mechanismus ähnlich zu oder wie Figur 11.

Allgemein kann es vorteilhaft sein, wenn zwischen einem Erwärmungs- oder Heizelement 10 und einem Sicherungselement oder Schmelzelement 9.6 ein direkter Kontakt vorliegt, wie z.B. bei den oben beschriebenen Varianten, bei welcher die Sicherung 9.6 auf Druck oder Scherung belastet wird. Als Material 9.6 wird beispielsweise ein Material verwendet, welches sich durch Zuführung von Wärme erweichen und/oder aufschmelzen lässt. Wie oben mehrfach erwähnt, kann auch ein Material verwendet werden, welches selbst als elektrischer Leiter dient oder elektrisch leitende Eigenschaften aufweist und somit selbst mittels Stromdurchfluss erwärmt und geschwächt oder geschmolzen werden kann.

Eine Schmelzauslösung aus Kunststoff sollte z.B. folgende Anforderungen erfüllen: Die Schmelzsicherung dient als Halteelement für den Auslösemechanismus während mehrerer Jahre. Deshalb ist auf einen kriechresistenen Kunststoff zu achten, der auch nicht spannungrissanfällig ist. Da sich das Kunststoffmaterial während der Lagerdauer nicht dehnen oder gar reissen sollte, ist darauf zu achten, dass die Streckspannung grösser als 45 MPa und die Streckdehnung kleiner als 5% beträgt.

Die Erweichungstemperaturen z.B. Vicat und HDT Temperaturen sollten im Bereich von 85°C bis 115°C liegen. Weiter sollten die verschiedenen Erweichungstemperaturen nahe beieinander liegen und der Kunststoff sollte amorph sein und eine niedrige Wärmekapazität besitzen, z.B. unter 2400 J/kgK

**Kunststoffe - Hintergrundinformationen**

Die Charakteristik des Temperaturverlaufs beim Schmelzen von teilkristallinen und amorphen Kunststoffen unterscheidet sich wie folgt:

Beim Schmelzen eines kristallinen Festkörpers muss am Schmelzpunkt Wärme zugeführt werden (latente Schmelzwärme), um die Kristallstruktur aufzulösen, die Temperatur kann währenddessen nicht erhöht werden. Beim amorphen Festkörper hingegen wird sämtliche zugeführte Energie verwendet, um die Temperatur des Stoffes zu erhöhen.

**Teilkristalline Kunststoffe**

Zunächst muss der Festkörper auf die Schmelztemperatur erwärmt werden, danach muss die Schmelzwärme hinzugefügt werden, um in die Schmelze überzugehen. Bei konstanten Druck und ohne Berücksichtigung von Verlusten (wie bspw. durch Konduktion, Stahlung oder Konvektion) gilt:

$$Q_{melt} = c \cdot m \cdot (T_m - T_a) + H_{fus} \; [J]$$

**Amorphe Kunsttoffe**

Bei amorphen Kunststoffen wird nur die Wärmekapazität bis zum Erreichen der Glasübergangstemperatur berücksichtigt. Bei konstanten Druck und ohne Berücksichtigung von Verlusten (wie bspw. durch Konduktion, Strahlung oder Konvektion) gilt:

$$Q_{melt} = c \cdot m \cdot \left(T_g - T_a\right) \; [J]$$

| | |
|---|---|
| Q | Wärmemenge |
| m | Masse des aufzuschmelzenden Kunststoffes [kg] |
| c | Spezifische Wärmekapazität [J/(kg·K)] |
| $H_{fus}$ | Schmelzwärme [J] |
| $T_m$ | Schmelztemperatur [C°] |
| $T_g$ | Glasübergangstemperatur [C°] |
| $T_a$ | Umgebungstemperatur [C°] |

$H_{fus}$[J]:    Schmelzwärme bezeichnet die Energiemenge, die benötigt wird, um eine Stoffprobe an ihrem Schmelzpunkt bei konstantem Druck (isobar) zu schmelzen, also vom festen in den flüssigen Aggregatzustand zu überführen.

c [J/(kg·K)]    Die **spezifische Wärmekapazität** ist eine thermo dynamische Stoffeigenschaft. Sie bemisst die Fähigkeit eines Stoffes, thermische Energie zu speichern.

**Bestimmen der erforderlichen Leistung zum Schmelzen eines Kunststoffes**

In Abhängigkeit der Zeit, die eingesetzt wird, um die Wärmemenge zum Schmelzen aufzubringen, kann die Leistung ermittelt werden.

$$P_{melt} = \frac{Q_{melt}}{t} \; \left[W = \frac{J}{s}\right]$$

Die folgende Tabelle zeigt bespielhaft eine Auswahl an Kunststoffen und die entsprechende Dauer, um mit einer bestimmten Leistung (ohne Berücksichtigung von Verlusten) ein bestimmtes Volumen an Kunststoff aufzuschmelzen respektive zu erweichen.
In der letzten Zeile ist die Dauer der Energiezufuhr bis der Kunststoff erweicht ist aufgeführt. Die Ergebnisse zeigen, dass teilkristalline Kunststoffe deutlich mehr Zeit und v.a. auch eine höhere Temperatur zum Aufschmelzen beanspruchen.

| Kunststoffe | | | PA | PBT 10% Glasfaser | PMMA | PC | PET | ABS |
|---|---|---|---|---|---|---|---|---|
| Verfügbare elektrische Leistung | P | W | 0.2 | keine Berücksichtigung von Wärmeverlusten | | | | |
| Ausgangstemperatur | T_init | °C | 23 | | | | | |
| Gefüge | | | teilkristallin | teilkristallin | amorph | amorph | teilkristallin (30-40%) | amorph |
| Volumen (Probe) | V | m3 | 3.0E-09 | 3.0E-09 | 3.0E-09 | 3.0E-09 | 3.0E-09 | 3.0E-09 |
| Dichte (Medium) | ρ | kg/m3 | 1440 | 1450 | 1190 | 1200 | 1400 | 1030 |
| Masse (Probe) | m | kg | 4.32E-06 | 4.35E-06 | 3.57E-06 | 3.60E-06 | 4.20E-06 | 3.09E-06 |
| Spezifische Wärmekapazität (Medium) | cs | J/(kgK) | 1200 | 1500 | 1500 | 1170 | 1500 | 1200 |
| Schmelzenergie (Medium) | H | J/kg | 213000 | 333000 | | | 145000 | |
| Schmelzwärme (Probe) | Qs | J | 9.20E-01 | 1.44855 | 0 | 0 | 0.609 | 0 |
| Schmelztemperatur (teilkristallin)/ Glasübergangstemp (amorph) | T_melt | °C | 220 | 223 | 110 | 148 | 250 | 95 |
| Temperaturdifferenz | ΔT | °C | 197 | 200 | 87 | 125 | 227 | 72 |
| Wärmeaufnahme (bisTS) | Q | J | 1.02 | 1.31 | 0.47 | 0.53 | 1.43 | 0.27 |
| Energie zum Wärmen und Schmelzen | Qtot | J | 1.94 | 2.75 | 0.47 | 0.53 | 2.04 | 0.27 |
| Dauer Energiezufuhr bis TS | Δt_TS | s | 5.11 | 6.53 | 2.33 | 2.63 | 7.15 | 1.33 |
| Dauer Energiezufuhr bis geschmolzen/ erweicht | Δt_melt | s | 9.71 | 13.77 | 2.33 | 2.63 | 10.20 | 1.33 |

**Angaben Datenblätter Kunststoffe**

Auf Kunststoff-Datenblättern sind unterschiedliche Angaben zu thermischen Eigenschaften zu finden. Meist sind entweder die HDT - heat deflection temperature oder die Vicat softening Temperatur angegeben. Die HDT-Temperaturen werden nach der Norm DIN EN ISO 75-1,-2,-3 (Vorläufer: DIN 53461) ermittelt. In der Norm wird zwischen den Varianten A und B und C unterschieden. Die Vicat softening Temperatur wird nach der Norm DIN EN ISO 306 (Vorläufer: DIN 53460) ermittelt.

**Geeignete Kunststoffe**

Die folgende Tabelle zeigt eine Auswahl an Kunststoffen. Die Auswahl ist nicht abschließend. Für die Auswahl wurden die oben erwähnten Punkte beachtet.

Zum Schmelzen eignen sich unter der Voraussetzung, dass das gegebene Heizelement eine Temperatur im Bereich von bspw. 120+/-5°C erreicht Kunststoffe mit einer Vicat Temperatur 85-115°C.

Sofern das Heizelement eine höhere Heizleistung erbringen kann, kommen weitere Kunststoffe in Frage.

EP 3 501 577 A1

| Werkstoff-Bezeichnung | Werkstoffeingenschaften thermisch | | | | | Werkstoffeingenschaften thermisch | | | | | vicat | | | HDT | | Δ HDT |
|---|---|---|---|---|---|---|---|---|---|---|---|---|---|---|---|---|
| Kunststofftyp | Tensile nodulus MPa | Yield stress MPa | Verhalten | Yield strain % | water absorbtion % | Tg | spezifische Wärmekapazität c [J/KgK] | Wärmeleitfähigkeit Fähigkeit [W/mK] | Temperatur der Wärmedaten (°C) | Dichte [kg/m3] | A/ Rate50 [°C] 10N | B/ Rate50 [°C] 50N | B/ Rate120 [°C] 50N | MethodA flexural stress s=1,8N/mm2 = const. | Method B: flexural stress s=0,45 N/mm2 = const. | Differenz zw. HDT A u. B [%] |
| ABS | 2400 | 4b | duktil | 2.6 | | | 1800 | 0.129 | 245 | 1040 | 102 | 99 | | 94 | 98 | 4.3 |
| ABS-GF | 5600 | 70 | spröde | 1.7 | | | | | | 1190 | | 105 | 106 | 104 | 107 | 2.9 |
| Cellulosics | 144b | 31 | duktil | 0.45 | | | | | | 1200 | | | | 75 | 8b | 17.3 |
| Copolyester | 1585 | 44 | dukbil | 7 | | 120 | 157b | 0.208 | | 1170 | | | | 92 | 109 | 18.5 |
| MABS | 2000 | 4b | duktil | 4 | 0.7 | | 2060 | 0.155 | 245 | 1080 | | 93 | | 90 | 94 | 4.4 |
| PC-ABS | 2100 | 50 | duktil | 4.5 | 0.7 | | 1960 | 0.157 | 260 | 1110 | | 113 | 115 | 99 | 120 | 21.2 |
| SAN | 3700 | 72 | spröde | 3 | 0.2 | | | 0.17 | | 1080 | | 10b | | 86 | 99 | 15.1 |
| SAN- GF35 | 12500 | 96 | spröde | 1 | | | | | | 1350 | | 105 | | 98 | 101 | 3.1 |
| SMMA | 3300 | 60 | spröde | 2.5 | 0.1 | | 2300 | 0.21 | 246 | 1080 | | 98 | 105 | 80 | 90 | 12.5 |

Besonders gut eignen sich verstärkte Kunststoffe. Sowie solche, bei denen der Unterschied zwischen HDT A und HDT B klein ist.

**Dimensionierung**

Die Dimensionierung von Bauteilen aus Kunststoff erfolgt gem. folgender Literatur:

- Gottfried W. Ehrenstein; Mit Kunststoffen konstruieren (ISBN 978-3-446-41322-1)

- Gunter Erhard; Konstruieren mit Kunststoffen (ISBN 3-446-22589-7)

Die zulässige Spannung der zu belastenden Struktur wird wie folgt ermittelt:

$$\sigma_{zul} = \frac{K}{S \cdot A}$$

$\sigma_{zul}$ = zulässige Spannung

$K$ = Kurzzeit - Festigkeltskennwert

$S$ = Sicherheitsfaktor

$A$ = Werkstoffabmindarungsfaktor (*Produkt aus dan untan genannten Warkstoffahmtndarungs -Einzelfaktoren*)

- Wertstoffabminderungs-Einzelfaktoren$A_T$ Festigkeitsabfall d. Temperatureinfluss geg. 20°

- $A_{st}$ Festigkeitsabfall durch Zeitstandbelastung

- $A_{dyn}$ Festigkeitsabfall aufgrund dyn. Belastung

- $A_A$ Alterungseinfluss

- $A_W$ Wassereinfluss (PA erheblich)

- $A_K$ Kerbeinfluss

- $A_{BN}$ Bindenaht

- $A_F$ Fertigungseinfluss (1.05-1.25)

- $A_{ex}$ Unsicherheit Ermittlung Kennwerte (Extrapolation oder ungenauer Kenntnis der Belastung)

**Rechenbeispiel zur Bestimmung von Abminderungs- und Sicherheitsfaktoren für ein amorphen Thermoplasten am Beispiel eines ABS (Acrylnitril-Butadien-Styrol-Copolymer):**

- Die folgende Tabelle weist die Werkstoffabminderungs- Einzelfaktoren am Beispiel **eines ABS (Acrylnitril-Butadien-Styrol-Copolymer)** aus:

| Mechanische Eigenschaften | ABS | ABS |
|---|---|---|
| Kurzzeit- Festigkeitskennwert K [N/mm2=Mpa] | 45 | 45 |
| Sicherheitsfaktor S | 2 | 2 |
| Abminderungsfaktoren (Ehrenstein S. 91) A | 2.622 | 5.182 |
| Abminderungsfaktoren (Ehrenstein S. 91) A inkl. Sicherheitsfaktor | 5.244 | 10.364 |
| **Zulässige Spannung [N/mm2]** | **8.581** | **4.342** |
| | | |
| **Erforderliche mindest. Querschnittsfläche [mm2] _max Kraft** | **0.655** | **1.294** |
| **Kraft** | **0.349** | **0.691** |
| **Erforderliche mindest. Querschnittsfläche [mm2] _max Kraft** | **0.139** | **0.275** |
| | | |
| Kantenlänge quadratischer Querschnitt [mm] | 0.81 | 1.14 |
| Durchmesser kreisrunder Querschnitt [mm] | 0.91 | 1.28 |
| Halten der Federkraft (direkt) | | |
| Erforderliche mindest. Querschnittsfläche [mm2] | 1.17 | 2.30 |
| | | |
| **Herleitung des Abminderungsfaktors** | | |
| A$_T$ Festigkeitsabfall d. Temperatureinfluss geg. 20° | 1.036 | 2.410 |
| **Temperatur** | **23** | **70** |
| k | 0.0117 | 0.0117 |
| | | |
| A$_{st}$ Festigkeitsabfall durch Zeitstandbelastung | 2 | 1.7 |
| Belastungsdauer | Jahre | 4 Tage |
| A$_{dyn}$ Festigkeitsabfall aufgrund dyn. Belastung | 1 | 1 |
| A$_A$ Alterungseinfluss | 1 | 1 |
| A$_W$ Wassereinfluss (PA erheblich) | 1 | 1 |
| A$_K$ Kerbeinfluss | 1 | 1 |
| A$_{BN}$ Bindenaht | 1 | 1 |
| A$_F$ Fertigungseinfluss (1.05-1.25) | 1.15 | 1.15 |
| Kenntnis der Belastung) | 1.1 | 1.1 |

Eine Belastung von ABS während 96h bei 70°C bspw. resultiert in einem höheren Abminderungsfaktor inkl. Sicherheitsfaktoren von 10.4 als die Belastung bei 23°C während mehreren Jahren (Abminderungsfaktor inkl. Sicherheitsfaktoren von 5.2).

Berücksichtig man folglich eine Reduktion der zulässigen Spannung um den Abminderungsinkl. Sicherheitsfaktor von 10.4, resultiert ein Wert für die zulässige Spannung zur mechanischen Belastung eines ABS- Bauteils von 4.3MPa.

Um die Gesamtverformung resp. Dehnung abzuschätzen, welche eine gewisse Last, bei gegebener Belastungsdauer und Umgebungstemperatur, auf eine Struktur eines Kunststoffbauteils bewirken kann, können isochrone Spannungsdehnungs- Diagramme od. Wertetabellen verwendet werden. Die Werte basieren auf Zugversuchen.

Die folgende Tabelle weist Spannungsdehnungskennwerte eines ABS Kunststoffes bei 23°C für unterschiedliche Belastungszeiträume auf:

| Stress-strain (isochronous) 23°C, Terluran® GP-22, ABS, INEOS Styrolution | | | | | | | | | |
|---|---|---|---|---|---|---|---|---|---|
| 10h | | 100h | | 1000h | | 10000h | | 100000h | |
| Strain in % | Stress in MPa | Strain in % | Stress in MPa | Strain in % | Stress in MPa | Strain in % | Stress in MPa | Strain in % | Stress in MPa |
| 0 | 0 | 0 | 0 | 0 | 0 | 0 | 0 | 0 | 0 |
| 0.1464 | 3 | 0.1608 | 3 | 0.1895 | 3 | 0.2472 | 3 | 0.3629 | 3 |
| 0.23 | 4.7 | 0.2534 | 4.7 | 0.3009 | 4.7 | 0.3971 | 4.7 | 0.5918 | 4.7 |
| 0.3142 | 6.4 | 0.3479 | 6.4 | 0.4166 | 6.4 | 0.5572 | 6.4 | 0.8443 | 6.4 |
| 0.3995 | 8.1 | 0.4448 | 8.1 | 0.5382 | 8.1 | 0.731 | 8.1 | 1.1284 | 8.1 |
| 0.4862 | 9.8 | 0.5452 | 9.8 | 0.668 | 9.8 | 0.9236 | 9.8 | 1.4556 | 9.8 |
| 0.5751 | 11.5 | 0.6505 | 11.5 | 0.809 | 11.5 | 1.142 | 11.5 | 1.8416 | 11.5 |
| 0.6669 | 13.2 | 0.7625 | 13.2 | 0.9654 | 13.2 | 1.3957 | 13.2 | 2.3082 | 13.2 |
| 0.7625 | 14.9 | 0.8836 | 14.9 | 1.1428 | 14.9 | 1.6975 | 14.9 | 2.8848 | 14.9 |
| 0.8634 | 16.6 | 1.0168 | 16.6 | 1.3481 | 16.6 | 2.064 | 16.6 | 3.6105 | 16.6 |
| 0.9516 | 18 | 1.1382 | 18 | 1.5447 | 18 | 2.4295 | 18 | 4.3558 | 18 |
| Quelle: https://www.campusplastics.com/campus/en/datasheet/Terluran%C2%AE+GP-22/INEOS+Styrolution/ 654/78717c93/SI?pos=0 | | | | | | | | | |

Die folgende Tabelle weist Spannungsdehnungskennwerte eines ABS Kunststoffes bei 60°C für unterschiedliche Belastungszeiträume auf:

| Stress-strain (isochronous) 60°C, Terluran® GP-22, ABS, INEOS Styrolution | | | | | | | | | |
|---|---|---|---|---|---|---|---|---|---|
| 10h | | 100h | | 1000h | | 10000h | | 10000h | |
| Strain in % | Stress in MPa | Strain in % | Stress in MPa | Strain in % | Stress in MPa | Strain in % | Stress in MPa | Strain in % | Stress in MPa |
| 0 | 0 | 0 | 0 | 0 | 0 | 0 | 0 | 0 | 0 |
| 0.1132 | 1.5 | 0.1559 | 1.5 | 0.2393 | 1.5 | 0.4022 | 1.5 | 0.7205 | 1.5 |
| 0.2058 | 2.7 | 0.2855 | 2.7 | 0.4419 | 2.7 | 0.7492 | 2.7 | 1.353 | 2.7 |
| 0.3016 | 3.9 | 0.4222 | 3.9 | 0.6604 | 3.9 | 1.1309 | 3.9 | 2.0604 | 3.9 |
| 0.4023 | 5.1 | 0.5695 | 5.1 | 0.9016 | 5.1 | 1.5613 | 5.1 | 2.8719 | 5.1 |
| 0.5098 | 6.3 | 0.7314 | 6.3 | 11741 | 6.3 | 2.0585 | 6.3 | 3.8253 | 6.3 |
| 0.6266 | 7.5 | 0.9132 | 7.5 | 1.489 | 7.5 | 2.6457 | 7.5 | 4.9693 | 7.5 |
| 0.7558 | 8.7 | 1.1214 | 8.7 | 1.8602 | 8.7 | 3.3524 | 8.7 | 6.3669 | 8.7 |
| 0.9011 | 9.9 | 1.3643 | 9.9 | 2.3051 | 9.9 | 4.2162 | 9.9 | 8.0984 | 9.9 |
| 1.0674 | 11.1 | 1.6518 | 11.1 | 2.8457 | 11.1 | 5.2843 | 11.1 | 10.2657 | 11.1 |
| 1.2953 | 12.5 | 2.0604 | 12.5 | 3.6335 | 12.5 | 6.8677 | 12.5 | 13.5171 | 12.5 |
| Quelle: https://www.campusplastics.com/campus/en/datasheet/Terluran%C2%AE+GP-22/INEOS+Styrolution/ 654/78717c93/SI?pos=0 | | | | | | | | | |

Das Beispiel der ABS Dimensionierung hat für die Belastung während 96h bei 70° eine zulässige Spannung von 4.3 MPa ergeben.

Aus den isochronen Spannungsdehnungs- Wertetabellen lässt sich nun abschätzen wie hoch die Gesamtverformung der Bauteil- Struktur beträgt sofern die Last für eine bestimmte Zeit bei einer bestimmten Temperatur beaufschlagt wird:

Bsp.

Lastfall1:    4.3 MPa bei 23°C während 100000h (~11.4 Jahre) -> Dehnung von ~ 0.5%

Lastfall2:    4.3 MPa bei 70°C während 100h -> Dehnung von ~ 0.55%

## Elektrisch leitfähiger Kunststoff

Elektrisch leitfähiger Kunststoff ist elektrisch leitfähig und vorzugsweise ein Thermoplast. Wird häufig für ESD-Kritische Bereiche (z.B. Verpackungen) eingesetzt.

Bei elektrisch leitfähigen Polymeren wird zwischen intrinsisch leitendenden und solchen, die aufgrund der Füllstoffe leitfähig werden, unterschieden.

Das Material kann zum Beispiel sehr feinen Ruß oder Aluminiumflocken enthalten. Durch die Größe der Partikel des Füllmaterials, Leitrusspartikel sind im Nanobereich anzusiedeln, ist es homogen im ganzen Bauteil verteilt. Die Leifähigkeit des Kunststoffes ist somit nicht davon abhängig, ob ein Stück am Rand oder in der Mitte kontaktiert wird. Das ermöglicht es auch sehr kleine Teile oder Geometrien auszubilden, die als Schmelzstelle wirken können, ohne Randeinflüsse zu erhalten.

Der Übergangswiderstand zwischen Kunststoffbauteil und Elektroden ist umso geringer, je inniger die Verbindung ist. Optimal ist eine möglichst große Kontaktfläche. Einen kleinen Übergangswiderstand erreicht man durch die Verbindung der Elektroden mit dem Kunststoff durch Verkleben, Verschweissen oder Verschrauben. Für den Übergangswiderstand spielt auch das Material und Oberflächenbeschaffenheit der Elektroden eine wichtige Rolle.

## Feststellung aus Messungen

Ein Element aus elektrisch leitendem Kunststoff kann auf einen gewünschten elektrischen Wert dimensioniert werden:

$$R = R_\square * \frac{L}{B}$$

    $R$: *Widerstandswert des Elements* [*Ohm*]
    $R_\square$: *Spezifischer Widerstand*[*Ohm* $\square$]
    *L: Abstand zwischen den Elektroden*[*m*]
    *B: Breite des Elements*[*m*]

Das elektrisch leitfähige Material Pre-Elec PP1830 weist einen spezifischen Widerstand von 5 Ohm cm bei 25 °C auf Unter Temperatureinwirkung verändert sich dieser Wert. Bei einer Temperatur von 80 °C beträgt er das Dreifache als bei 25 °C. Bei einer Temperatur von 140 °C weist er wieder denselben Wert wie bei 25 °C auf.

Der Widerstandswert verändert sich, die Temperatur ausgeblendet, nur wenig. Selbst wenn mit einem Gegenstand eingestochen wird, bleibt die Leitfähigkeit weitgehend erhalten. Eine verschweisste Kontaktierung bleibt stabil und ist auch nach dem Erkalten noch intakt.

Einer der Vorteile des elektrisch leitenden Kunststoffs ist, dass ein Bauteil gleichzeitig als Heizung und zu schmelzendes Element wirkt. Dies ermöglicht einen kompakteren Aufbau, zudem werden dadurch die Verluste durch Wärmestrahlung und Wärmeübergang reduziert. Weiter bietet der elektrisch leitende Kunststoff eine hohe Gestaltungsfreiheit bei der Geometrie und kann effizient durch Spritzguss hergestellt werden.

## Heizdraht

Idee: Das zu schmelzende Element wird mit einer metallischen Spule umgeben. Aufgrund der hohen Leitfähigkeit des Metalls wird ein Draht lang, wenn er einen gewünschten elektrischen Widerstandswert erreichen soll. Unteranderem sind folgende metallische Materialien grundsätzlich geeignet für eine solche Anwendung: Aluminium, Zink, Zinn, Kupfer und Wolfram.

Die Vorteile dieser Variante ist, dass das Drahtmaterial gut verfügbar und vergleichsweise preiswert ist. Weiter kann die zu erweichende Stelle direkt mit dem Draht umwickelt werden, oder der Draht kann beim Kunststoffspritzgiessen direkt eingelegt werden. Dadurch ist der Abstand zwischen Wärmequelle und zu erwärmenden Querschnitt sehr klein und Toleranzen können aufgehoben werden.

Der spezifische Widerstand des Materials sollte so gross sein, dass sich eine passende Länge des Drahts ergibt. Die Länge des Drahts sollte in der Grössenordnung von 10 mm sein. Für den Betrieb mit einem Akku sollte der Widerstandswert zwischen 10 bis 20 Ohm liegen. Mit diesen Werten kann eine praktikable Heizleistung erzeugt werden, um den Draht auf die gewünschte Temperatur zu erhitzen.

Spezifischer Widerstandswerte für geeignete Materialien

| Material | Spez. Widerstand ϱ 20 °C (Ohm*mm2/m) |
|----------|--------------------------------------|
| Aluminium | 0.027 |
| Zinn | 0.11 |
| Wolfram | 0.055 |
| Zink | 0.061 |
| Kupfer | 0.0178 |

$$R = \frac{\rho * l}{A}$$

R: Widerstandswert des Drahts [*Ohm*]

$\rho$: Spezifischer Widerstand $[Ohm * \frac{mm2}{m}]$

l: Länge des Drahts[*m*]

A: Querschnitt des Drahts[*mm*2]

Beispielrechnung für Zinn:
Spez. Widerstand   0.11 Ohm mm2/m
Querschnitt        0.01 mm2
Länge              0.01 m
Widerstand         R = 0.11 Ohm

**Elektrische Leistung**

Die Energiequelle in der Patchpumpe kann idealerweise 0.5 Watt während mehreren Sekunden liefern. Dies hat Einfluss auf die Gestaltung und Auslegung der Wärmequelle. Das Heizelement resp. die Wärmequelle muss mit der zur Verfügung stehenden elektrischen Leistung das Medium erwärmen und zum wegweichen/wegfließen bringen.

Als Wärmequelle kommen verschiedene Medien infrage.

**SMD Widerstand**

In der Elektronik eingesetzte Widerstände für die Oberflächenmontage sind in unterschiedlich grossen Bauformen erhältlich. Sie sind sehr widerstandsfähig und der gleiche Widerstand kann während vielen Schmelzvorgängen verwendet werden. Bedingt durch ihre Bauart und Kontaktierung ist die Wärmeableitung auf die anschließenden Leiter relativ hoch. Ihr Vorteil ist, dass sie sehr temperaturresistent sind. Die Temperaturresistenz ist durch den Herstellprozess gegeben - die resistive Paste wird bei 850°C eingebrannt. Dadurch ist der Widerstand auch sehr robust gegenüber mechanischer Beschädigung. Zudem sind solche Widerstände Massenware, wodurch sie sehr preiswert sind.

**Customized Bauform Widerstand**

Nach dem Prinzip des SMD-Widerstand könnte eine spezifische Bauform des Substrat hergestellt werden. Diese wird an der gewünschten Stelle mit einer Widerstandsschicht ausgestattet. Der Vorteil dieser Variante liegt, neben der Hitzeresistenz, der Robustheit gegenüber mechanischer Beschädigung, vor allem in der geringeren Wärmekapazität und der Möglichkeit die Geometrie individuell zu gestalten, womit die Effizienz deutlich erhöht werden kann.

**Widerstandsdruck auf Leiterplatten (gedruckte Polymer)**

Die Technologie des Widerstandsdrucks auf Leiterplatten wird häufig in Bedienelementen wie Taster und Drehpotentiometer in Bedienelementen eingesetzt.

Carbonlack kann für direkt auf Leiterplatten gedruckte Widerstände verwendet werden. Hierzu wird die Lücke zwischen zwei Leiterbahnen durch eine Fläche aus Carbon-Leitlack überdeckt. In diesem Bereich ist die Leiterplatte frei von Lötstopplack. Der Widerstand ist gleich dem spezifischen Flächenwiderstand [W (je square)], multipliziert mit dem Verhältnis von Abstand zu Breite des Carbon-Lacks:

Es stehen Carbonpasten mit einem Flächenwiderstand von 10 Ohm bis 1000 kOhm zur Verfügung. Unter Ausnutzung verschiedener Lack-Geometrien (z. B. Meander) können Widerstände von wenigen Ohm bis zu mehreren 10 kOhm erzeugt werden. Die Toleranz des Widerstands in der Endanwendung liegt ohne Laserabgleich bei ca. 30% und mit Laserabgleich bei 5 %. Die Schichtdicke der Paste beträgt 20 $\mu$m. Die minimale Breite der Strukturen z.B. der Meander beträgt 0.2 mm

Die Vorteile dieser Variante liegen in der sehr geringen Wärmekapazität, was zu einer sehr schnellen Erwärmung führt. Weiter kann die Form des Widerstandes sehr frei gewählt werden und die Herstellung ist sehr kostengünstig.

**Widerstandsdruck auf Substrat mit Aerosol-Jet Druckverfahren**

Bedruckung von fast beliebigen Substratmaterialien und Formen mit Widerstandspasten. Es stehen verschiedene Widerstandspasten zur Auswahl.

Die Vorteile liegen darin, dass ein Substrat gewählt werden kann, welches eine hohe Wärmeisolation und geringe Wärmekapazität aufweist. Ein weiterer Vorteil ist die Möglichkeit, die Heizfläche drei dimensional gestalten zu können und dadurch die Wärmekopplung zum schmelzbaren Element zu erhöhen. Die Technologie ermöglicht das Herstellen von sehr kleinen Strukturen, was die eine geringe Wärmekapazität ermöglicht.

**Bezugszeichenliste**

| | |
|---|---|
| 1 | Kanülengehäuse |
| 1.1 | Sperr- oder (Rück-)Halteelement oder Auslöseelement Inserter |
| 1.1a | Antriebsfläche oder Schräge |
| 1.2 | Reverser-Führung, Ablaufsteuerung, Kanülengehäusefinger |
| 1.2a | schmaler Entkoppelbereich |
| 1.2b | breiter Koppelbereich |
| 2 | Soft-Kanülen-Halter |
| 2.1 | Hintergriff oder Auslöseelement Reverser, Koppelelement |
| 2.2 | Halterung Softkanüle |
| 2.3 | Öffnung für Ablaufsteuerung und Führung |
| 2.4 | Anlagefläche, Insertionsfederanschlag |
| 2.5 | Anlagefläche, Hart-Kanülen-Halter-Anschlag |
| 3 | Hart-Kanülen-Halter |
| 3.1 | Hintergriff Reverser oder Gegenkoppelelement |
| 3.2 | Halterung Hartkanüle |
| 4 | Insertionsfeder |
| 5 | Rückzugsfeder |
| 6 | Kanülenführung |
| 6.1 | Kanülenführung |
| 7 | Softkanüle |
| 8 | Hartkanüle, Stahlkanüle |
| 8.1 | Kanülenschleife |
| 9 | Sicherung(selement) oder Schmelzsicherung |
| 9.1 | Verbindungselement |
| 9.2a, b | Halterung |
| 9.3 | Aufweichelement, Schmelzelement |
| 9.4 | Aufweichelement, Schmelzelement |
| 9.5 | Halteelement, Aufweichelement, Schmelzelement |
| 9.6 | Aufweichelement, Schmelzelement, schmelzbares Medium |

| 10 | Erwärmungs- oder Heizelement |
|---|---|
| 10a, b | Ausfräsung |
| 10c | elektrischer Kontakt |
| 11 | Pumpe, Reservoir |
| 12 | Haftfläche (Patch Pumpe) |
| 13 | Batterie |
| 14 | Führung, Hohlzylinder |
| 14a, b | Abflussöffnung |
| 15 | Stößel |
| 16 | Folie |
| 17 | Haftfläche oder Klebefläche; Halteelement |
| 18 | Basiselement |
| 19 | aufgeklebte Struktur |

**Patentansprüche**

1. Insertionsmechanismus für eine Kanüle (7, 8) mit:

   einem Kanülengehäuse (1), relativ zu welchem die Kanüle (7, 8) verschoben werden kann;
   mindestens einem Kanülenhalter (2.2, 3.2), an welchem die Kanüle (7, 8) befestigbar oder befestigt ist und welcher relativ zum Kanülengehäuse (1) bewegbar ist;
   einem Insertions-Energiespeicher (4) zum Erzeugen einer Insertionskraft auf den mindestens einen Kanülenhalter (2.2, 3.2);
   einem Halteelement (1.1), welches den mindestens einen Kanülenhalter (2.2, 3.2) gegen die vom Insertions-Energiespeicher (4) erzeugte Kraft relativ zum Kanülengehäuse (1) halten kann; und
   einer Sicherung (9), welche das Halteelement (1.1) im Sicherungszustand in Sperrstellung hält und im ausgelösten Zustand das Halteelement (1.1) freigibt, sodass das Halteelement (1.1) in Freigabestellung bewegt werden kann und sich der mindestens eine Kanülenhalter (2.2, 3.2), angetrieben durch den Insertions-Energiespeicher (4), relativ zum Kanülengehäuse (1) bewegen kann, um eine Insertionsbewegung oder Insertion auszulösen oder durchzuführen,
   wobei die Sicherung (9) oder ein Sicherungselement oder ein Teil der Sicherung ein aufweichbares oder schmelzbares Material umfasst, welches mit einem Heizelement (10) von einer Seite und/oder an einer Fläche erwärmt und aufgeweicht und/oder in seiner mechanischen Trag- oder Haltefähigkeit geschwächt oder durchtrennt oder zumindest teilweise oder vollständig geschmolzen werden kann.

2. Insertionsmechanismus nach Anspruch 1, wobei die Sicherung (9) oder das Sicherungselement nur von einer einzigen Seite oder an einer einzigen Fläche erwärmt wird.

3. Insertionsmechanismus nach einem der vorhergehenden Ansprüche, wobei die Sicherung (9) oder das Sicherungselement nicht umfassend oder nicht umschließend erwärmt wird.

4. Insertionsmechanismus nach einem der vorhergehenden Ansprüche, wobei das Halteelement (1.1) stoffschlüssig und/oder formschlüssig mit der Sicherung (9) verbunden ist.

5. Insertionsmechanismus nach einem der vorhergehenden Ansprüche, wobei das schmelzbare oder aufweichbare Material so mit dem Halteelement (1.1) gekoppelt ist, dass es in einem Sicherungszustand auf Zug beansprucht wird; oder so mit dem Halteelement (1.1) gekoppelt ist, dass es in einem Sicherungszustand auf Druck beansprucht wird.

6. Insertionsmechanismus nach einem der vorhergehenden Ansprüche, wobei das Sicherungselement (9) eine Verjüngung aufweist, welche im Erwärmungs- oder Erhitzungsbereich des Sicherungselementes (9) liegt.

7. Insertionsmechanismus für eine Kanüle (7, 8) mit:

   einem Kanülengehäuse (1), relativ zu welchem die Kanüle (7, 8) verschoben werden kann;
   mindestens einem Kanülenhalter (2.2, 3.2), an welchem die Kanüle (7, 8) befestigbar oder befestigt ist und welcher relativ zum Kanülengehäuse (1) bewegbar ist;

einem Insertions-Energiespeicher (4) zum Erzeugen einer Insertionskraft auf den mindestens einen Kanülenhalter (2.2, 3.2);

einem Halteelement (1.1), welches den mindestens einen Kanülenhalter (2.2, 3.2) gegen die vom Insertions-Energiespeicher (4) erzeugte Kraft relativ zum Kanülengehäuse (1) halten kann; und

einer Sicherung (9), welche das Halteelement (1.1) im Sicherungszustand in Sperrstellung hält und im ausgelösten Zustand das Halteelement (1.1) freigibt, sodass das Halteelement (1.1) in Freigabestellung bewegt werden kann und sich der mindestens eine Kanülenhalter (2.2, 3.2), angetrieben durch den Insertions-Energiespeicher (4), relativ zum Kanülengehäuse (1) bewegen kann, um eine Insertionsbewegung oder Insertion auszulösen oder durchzuführen,

wobei die Sicherung (9) oder ein Sicherungselement ein von dem Halteelement (1.1) auf Druck belastetes Element (9.4; 15) ist, welches gegen ein Abstützelement (10; 9.5) drückt,

wobei die Sicherung des Halteelements (1.1) durch die Sicherung (9) oder das Sicherungselement durch Erwärmung gelöst werden kann.

8. Insertionsmechanismus nach Anspruch 7, wobei die Sicherung (9) oder ein Sicherungselement durch Erwärmen aufgeweicht oder geschmolzen oder geschwächt werden kann und/oder das Sicherungselement aus einem Material besteht, das den vom Halteelement (1.1) ausgeübten Druck im erwärmten Zustand nicht mehr halten kann und so das Halteelement (1.1) freigibt.

9. Insertionsmechanismus nach einem der zwei vorhergehenden Ansprüche, wobei die Sicherung (9) oder das Sicherungselement dadurch gelöst wird, dass das Abstützelement (9.5) oder zumindest ein Teil davon erwärmt oder erweicht oder geschmolzen wird, sodass das auf Druck belastete Element (15) es dem Halteelement (1.1) ermöglicht, in Freigabestellung zu gelangen.

10. Insertionsmechanismus nach einem der drei vorhergehenden Ansprüche, wobei das auf Druck belastete Element (9.4; 15) ein längliches Element oder stabförmiges Element ist.

11. Insertionsmechanismus nach einem der vier vorhergehenden Ansprüche, wobei das auf Druck belastete Element (9.4; 15) in mindestens einem Führungselement (14) oder einer Führung geführt ist, welche eine Verschiebung des auf Druck belasteten Elements (9.4; 15) ermöglicht.

12. Insertionsmechanismus nach einem der fünf vorhergehenden Ansprüche, wobei das Abstützelement (9.5) aus Lot oder ein Lötmittel ist.

13. Insertionsmechanismus nach einem der vorhergehenden Ansprüche, wobei die Sicherung (9) oder ein Sicherungselement aus einem leitfähigen Material oder leitfähigem Kunststoff besteht.

14. Insertionsmechanismus nach einem der vorhergehenden Ansprüche, wobei die Kanüle eine Softkanüle (7), eine Hartkanüle (8), oder eine eine Hartkanüle (8) umgebende Softkanüle (7) ist oder eine von einer Hartkanüle (8) umgebene Softkanüle (7) ist.

15. Insertionsmechanismus nach einem der vorhergehenden Ansprüche, wobei der Insertions-Energiespeicher (4) eine Druckfeder ist, welche eine Druckkraft auf den mindestens einen Kanülenhalter (2.2, 3.2) ausübt; oder eine Zugfeder ist, welche eine Zugkraft auf den mindestens einen Kanülenhalter (2.2, 3.2) ausübt.

16. Insertionsmechanismus nach einem der vorhergehenden Ansprüche mit einem Rückzugs-Energiespeicher (5) zum Zurückziehen einer Hartkanüle (8).

17. Insertionsmechanismus nach einem der vorhergehenden Ansprüche mit einem Erwärmungs- oder Heizelement (10), das an oder bei der Sicherung oder dem Sicherungselement (9) oder als Abstützelement angeordnet ist.

18. Verabreichungsvorrichtung zur Verabreichung einer Substanz mit einem Insertionsmechanismus nach einem der vorhergehenden Ansprüche und einem Reservoir für die Substanz und/oder einer Pumpe, welche mit dem proximalen Ende einer Kanüle (8) verbunden ist.

19. Verfahren zum automatischen Ausstoßen einer Kanüle (7, 8) mit einem Insertionsmechanismus nach einem der vorhergehenden Ansprüche, wobei:

das Sicherungselement (9) durch ein elektrisches Signal oder durch Stromfluss durch ein flächiges Heizelement (10), welches auf einer Seite des Sicherungselements (9) angeordnet ist, gelöst wird;

das Sicherungselement (9) erwärmt und geschwächt oder aufgeweicht oder geschmolzen wird und das Halteelement (1.1) freigibt; und

das Halteelement (1.1) durch die Kraft des Insertions-Energiespeichers (4) oder der Insertions-Feder in eine Freigabeposition gedrückt oder gezogen wird, wonach eine Kanülen-Insertionsbewegung begonnen wird oder werden kann.

20. Verfahren zum automatischen Ausstoßen einer Kanüle (7, 8) mit einem Insertionsmechanismus nach einem der vorhergehenden Ansprüche, wobei:

das Sicherungselement (9) durch ein elektrisches Signal gelöst wird;

das Sicherungselement (9) das Halteelement (1.1) freigibt; und

das Halteelement (1.1) durch die Kraft des Insertions-Energiespeichers (4) oder der Insertions-Feder in eine Freigabeposition gedrückt oder gezogen wird, wonach eine Kanülen-Insertionsbewegung begonnen wird oder werden kann.

21. Verfahren zum automatischen Ausstoßen oder Verschieben einer Kanüle (7, 8) nach Anspruch 19 oder 20, wobei das elektrische Signal ein Stromfluss durch ein Heizelement (10) und/oder durch das Sicherungselement (9) ist.

Fig. 1A

Fig. 1B

Fig. 3

Fig. 2

Fig. 4

Fig. 5

Fig. 6

1.2

1.2b

Fig. 7A

2.1

2.1

1.2a

2.1

2.1

2.1

Fig. 7B

Fig. 8A

Fig. 8B

Fig. 8C

Fig. 8D

Fig. 8E

Fig. 8G

Fig. 8J

Fig. 8I

Fig. 8F

Fig. 8H

Fig. 8K

Fig. 8L

Fig. 8M

Fig. 8N

2.2  5  3.2

7  4

Fig. 8O

Fig. 9B

Fig. 9E

Fig. 9A

Fig. 9D

Fig. 9C

9.2a

9.2b

9.3

Fig. 9F

Fig. 9G

Fig. 9H

Fig. 9I

Fig. 9J

Fig. 9L

Fig. 9O

Fig. 9N

Fig. 9K

Fig. 9M

Fig. 10A

Fig. 10B

Fig. 10C

Fig. 10D

Fig. 10E

Fig. 10G

Fig. 10J

Fig. 10F

Fig. 10I

Fig. 10H

1.1a

Fig. 10O

Fig. 10L

Fig. 10N

Fig. 10K

Fig. 10M

Fig. 11A

Fig. 11B

Fig. 11C

Fig. 11D

Fig. 11E

Fig. 11G

Fig. 11F

Fig. 11J

Fig. 11I

Fig. 11H

Fig. 11O

Fig. 11L

Fig. 11N

Fig. 11K

Fig. 11M

Fig. 12B

Fig. 12E

Fig. 12A

Fig. 12C

Fig. 12D

Fig. 12G

Fig. 12F

16

16c

Fig. 12J

16

Fig. 12I

Fig. 12H

1.1

1.1

Fig. 12K

Fig. 12L

Fig. 12M

Fig. 12N

Fig. 12O

Fig. 13A

Fig. 13B

Fig. 13C

Fig. 13D

Fig. 13E

Fig. 13G

Fig. 13F

Fig. 13J

Fig. 13I

Fig. 13H

Fig. 13O

Fig. 13L

Fig. 13N

Fig. 13K

Fig. 13M

Fig. 14

1.1

9.6

10

18

Fig. 15

1.1

9.6

10

18

Fig. 16

Fig. 17

Fig. 18

Fig. 19

Fig. 20

Fig. 21

Fig. 22

Fig. 23

Fig. 24

Fig. 25A

Fig. 25B

Fig. 26

Fig. 27

**Europäisches Patentamt**
**European Patent Office**
**Office européen des brevets**

## EUROPÄISCHER TEILRECHERCHENBERICHT

nach Regel 62a und/oder 63 des Europäischen Patentübereinkommens. Dieser Bericht gilt für das weitere
Verfahren als europäischer Recherchenbericht.

**Nummer der Anmeldung**

EP 17 20 9764

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| X | EP 2 762 183 A1 (TERUMO CORP [JP]) 6. August 2014 (2014-08-06) * Absätze [0111] - [0140], [0313] - [0318]; Abbildungen 1-15,32 * | 1-6,19, 21 | INV. A61M5/32 A61M5/142 A61M5/158 |
| X | WO 2012/143434 A2 (SANOFI AVENTIS DEUTSCHLAND [DE]; CROSS JOHN DAVID [GB]; BAINTON MICHAE) 26. Oktober 2012 (2012-10-26) * Seite 30, Zeile 4 - Seite 31, Zeile 2; Abbildungen 26-28 * | 1-6,19, 21 | |
| A | WO 99/02208 A1 (PETS N PEOPLE LTD [IL]; GORDON TAL [IL]) 21. Januar 1999 (1999-01-21) * Seite 11, Zeile 6 - Seite 14, Zeile 21; Ansprüche 7-10; Abbildungen 9-21 * | 1-6,19, 21 | |
| A | US 2005/222537 A1 (DINSMOOR DAVID A [US] ET AL) 6. Oktober 2005 (2005-10-06) * Absätze [0071] - [0079], [0082], [0089]; Ansprüche 9, 10, 35; Abbildungen 8-17 * | 1-6,19, 21 | RECHERCHIERTE SACHGEBIETE (IPC) A61M |

### UNVOLLSTÄNDIGE RECHERCHE

Die Recherchenabteilung ist der Auffassung, daß ein oder mehrere Ansprüche, den Vorschriften des EPÜ nicht entspricht bzw. entsprechen, so daß nur eine Teilrecherche (R.62a, 63) durchgeführt wurde.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| München | 31. Juli 2018 | Messmer, Melitta |

EPO FORM 1503 03.82 (P04E09)

Europäisches
Patentamt

European
Patent Office

Office européen
des brevets

**UNVOLLSTÄNDIGE RECHERCHE
ERGÄNZUNGSBLATT C**

**Nummer der Anmeldung**

EP 17 20 9764

Vollständig recherchierbare Ansprüche:
      1-6, 19

Unvollständig recherchierte Ansprüche:
      21

Nicht recherchierte Ansprüche:
      7-18, 20

Grund für die Beschränkung der Recherche:

Wie vom Anmelder auf die Mitteilung nach Regel 62a(1) EPÜ (am 13.07.2018) beantragt, wurden die Ansprüche 1-6 sowie 19 und 21 recherchiert.

**ANHANG ZUM EUROPÄISCHEN RECHERCHENBERICHT
ÜBER DIE EUROPÄISCHE PATENTANMELDUNG NR.**

EP 17 20 9764

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-07-2018

| Im Recherchenbericht angeführtes Patentdokument | Datum der Veröffentlichung | Mitglied(er) der Patentfamilie | Datum der Veröffentlichung |
|---|---|---|---|
| EP 2762183 A1 | 06-08-2014 | CN 103826678 A<br>EP 2762183 A1<br>US 2014228759 A1<br>WO 2013047855 A1 | 28-05-2014<br>06-08-2014<br>14-08-2014<br>04-04-2013 |
| WO 2012143434 A2 | 26-10-2012 | CA 2832957 A1<br>EP 2699283 A2<br>JP 2014514097 A<br>US 2014058333 A1<br>WO 2012143434 A2 | 26-10-2012<br>26-02-2014<br>19-06-2014<br>27-02-2014<br>26-10-2012 |
| WO 9902208 A1 | 21-01-1999 | AU 8239398 A<br>CA 2295516 A1<br>EP 0994741 A1<br>JP 2001509424 A<br>US 6349232 B1<br>US 2002065479 A1<br>US 2006037557 A1<br>US 2008011240 A1<br>US 2010072222 A1<br>US 2011139088 A1<br>WO 9902208 A1 | 08-02-1999<br>21-01-1999<br>26-04-2000<br>24-07-2001<br>19-02-2002<br>30-05-2002<br>23-02-2006<br>17-01-2008<br>25-03-2010<br>16-06-2011<br>21-01-1999 |
| US 2005222537 A1 | 06-10-2005 | DE 102005013612 A1<br>FR 2868280 A1<br>US 2005222537 A1<br>US 2010217368 A1 | 10-11-2005<br>07-10-2005<br>06-10-2005<br>26-08-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9220838 B2
- CH 0006117
- CH 0006317
- CH 0006217
- CH 0020817

**In der Beschreibung aufgeführte Nicht-Patentliteratur**

- **GOTTFRIED W. EHRENSTEIN.** *Mit Kunststoffen konstruieren,* ISBN 978-3-446-41322-1
- **GUNTER ERHARD.** *Konstruieren mit Kunststoffen,* ISBN 3-446-22589-7